(19)

Europäisches Patentamt
European Patent Office
Office européen des brevets

(11) EP 2 420 222 B1

(12) EUROPÄISCHE PATENTSCHRIFT

(45) Veröffentlichungstag und Bekanntmachung des Hinweises auf die Patenterteilung:
14.12.2016 Patentblatt 2016/50

(51) Int Cl.:
A45D 31/00 (2006.01)      A61Q 3/02 (2006.01)
A61K 8/81 (2006.01)       A61K 8/19 (2006.01)

(21) Anmeldenummer: 10008669.3

(22) Anmeldetag: 19.08.2010

(54) Nagellack mit samtiger Haptik

Nail varnish with velvet feel

Vernis à ongle doté d'un toucher velours

(84) Benannte Vertragsstaaten:
AL AT BE BG CH CY CZ DE DK EE ES FI FR GB GR HR HU IE IS IT LI LT LU LV MC MK MT NL NO PL PT RO SE SI SK SM TR

(43) Veröffentlichungstag der Anmeldung:
22.02.2012 Patentblatt 2012/08

(73) Patentinhaber: Eckart GmbH
91235 Hartenstein (DE)

(72) Erfinder:
• Schmidt, Ulrich
91217 Hersbruck (DE)
• Krüger, Peter
89231 Neu-Ulm, Ludwigsfeld (DE)
• Gerstacker, Michaela
91245 Simmelsdorf (DE)
• Kurz, Sonja
92259 Neukirchen (DE)
• Mendler, Barbara
88709 Meersburg (DE)

(74) Vertreter: Walcher, Armin et al
Louis, Pöhlau, Lohrentz
Patentanwälte
Postfach 30 55
90014 Nürnberg (DE)

(56) Entgegenhaltungen:
EP-A1- 0 479 669      WO-A1-2009/129909
DE-A1- 2 345 560      JP-A- 11 302 563
JP-A- 63 275 512      US-A- 3 407 160
US-A- 5 330 750

• "Waxes for Personal Care Applications.", , 1. November 2007 (2007-11-01), XP002617147, Gefunden im Internet: URL:http://www.clariant.com/C125720D002B96 3C/30C9C69F60864924C125739B0047B1C0/$FIL E/ DP5025E_1107_BR_WaxesforPersonalCareAppl ic ations.pdf [gefunden am 2011-01-17]

**Beschreibung**

Die vorliegende Erfindung betrifft einen Nagellack, welcher sich nach Applikation und Trocknung durch eine außergewöhnliche Kombination von haptischen und optischen Eigenschaften auszeichnet, ein Verfahren zu dessen Herstellung, die Verwendung von wenigstens einem mikronisierten Wachs in einem Nagellack sowie einen mit dem Nagellack versehenen Gegenstand.

Um vom Verbraucher in der Vielzahl der angebotenen Nagellacke überhaupt wahrgenommen zu werden, sind nicht nur Nagellacke gefragt, welche den jeweils aktuellen Trendfarben folgen, sondern auch solche, welche originelle, neu- oder einzigartige Effekte besitzen.

Aus US 4,822,423 ist Siliziumdioxid als Mattierungsmittel für einen Nagellack bekannt. Siliziumdioxid mit einer Partikelgröße von 0,01 bis 30 $\mu$m wird hier in einem Anteil von 1 bis 10 Gew.-% der Nagellackformulierung zugesetzt, um einen glanzlosen Lackfilm auf dem Nagel zu erhalten.

Fingernägel können gemäß US 2009/0126316 A1, WO 2007/039832 A2 oder WO 2009/097517 A1 mit einem sicht- und/ oder fühlbaren Reliefeffekt versehen werden. Um diesen Reliefeffekt zu erzeugen, muss eine Beschichtung aus mehreren Schichten aufgebracht werden, wobei mindestens eine Schicht sphärische Partikel oder Fasern aufweist.

(Künstliche) Fingernägel mit Reliefeffekt können nach dem in US 6,367,484 B1 beschriebenen Verfahren erhalten werden. Hierzu wird der Fingernagel zunächst mit einer filmbildenden Schicht versehen, auf die in dieser unlösliche, sphärische oder ovale Partikel aufgebracht werden. Diese Partikel verleihen dem Fingernagel neben dem Relief- auch einen "soft feel"-Effekt. Für einen guten Halt der sphärischen oder ovalen Partikel können diese mittels einer zweiten filmbildenden Schicht auf der ersten fixiert werden. Als sphärische Partikel werden bevorzugt Glaskügelchen eingesetzt. Das beschriebene Verfahren ist nicht nur auf Fingernägel beschränkt, sondern kann auch auf Haut, Lippen oder Haaren angewendet werden.

Eine weitere Möglichkeit einer kosmetischen Formulierung, u.a. einem Nagellack, einen "soft touch"-Effekt zu verleihen, kann, wie in US 5,612,021 beschrieben, durch Zusatz eines Fullerens oder einer Mischung von Fullerenen erfolgen.

Ein künstlicher Fingernagel enthaltend körnige Materialien, wie Glaskügelchen, Salz oder Zucker, welche dem künstlichen Fingernagel eine strukturierte Oberfläche verleihen, wird in US 4,407,310 beschrieben.

Nagellacke enthaltend Mikrokugeln sind aus der WO 00/27347 A1 bekannt. In Abhängigkeit des Durchmessers der Mikrokugeln kann einem mit diesem Nagellack lackierten Fingernagel ein unterschiedliches Aussehen verliehen werden. Feinere Mikrokugeln dienen beispielsweise der Glanzsteigerung (Durchmesser: 1 bis 12 $\mu$m) oder können als Füllstoff zum Ausgleich von Unebenheiten des Fingernagels (Durchmesser: 1 bis 40 $\mu$m) verwendet werden. Größere Mikrokugeln (Durchmesser: 50 bis 150 $\mu$m) können einem mit diesem Nagellack lackierten Fingernagel Struktur oder ein mattes Aussehen geben.

DE 2345560 offenbart einen Nagellack, der aminmodifizierten Montmorillonitton enthält.

Aufgabe der vorliegenden Erfindung ist es, einen Nagellack bereitzustellen, welcher sich nach Applikation und Trocknung durch eine außergewöhnliche Kombination von haptischen und optischen Eigenschaften auszeichnet. Der Nagellack soll einem hiermit lackierten (künstlichen) Fingernagel eine samtige Haptik verleihen, welcher vom Anwender als angenehm wahrgenommen wird. Gleichzeitig soll ein attraktives optisches Erscheinungsbild erzielt werden.

Der applizierte Nagellack soll weiterhin eine ausreichende mechanische Stabilität aufweisen und nicht bereits durch gängige Haus- und/ oder Büroarbeit beschädigt werden. Außerdem soll der applizierte Nagellack über einen möglichst langen Zeitraum in optisch ansprechender Form erhalten bleiben. Aufgabe der Erfindung ist es weiterhin, ein Verfahren zu Herstellung dieses Nagellacks zur Verfügung zu stellen.

Die der Erfindung zugrundeliegende Aufgabe wurde durch Bereitstellung eines Nagellacks gemäß Anspruch 1 gelöst.

Bevorzugte Weiterbildungen des erfindungsgemäßen Nagellacks sind in den abhängigen Ansprüchen 2 bis 4 angegeben.

Weiterhin wurde die Aufgabe durch Bereitstellung eines Verfahrens zur Herstellung des erfindungsgemäßen Nagellacks gelöst, wobei das Verfahren wenigstens folgenden Schritt umfasst: Vermengen einer Nagellackbasis, wenigstens eines mikronisierten Wachses und wenigstens eines Farbmittels unter Bereitstellung eines Nagellacks.

Hierbei ist die Zugabereihenfolge unerheblich. Bevorzugt werden die nicht in der Nagellackbasis löslichen Komponenten des erfindungsgemäßen Nagellacks, mikronisierte(s) Wachs(e) und Pigment(e) nacheinander oder als Mischung einer mit einem löslichen Farbstoff eingefärbten oder nicht eingefärbten Nagellackbasis zugefügt. In der Praxis hat es sich bewährt, plättchenförmige Effektpigmente im letzten Arbeitsschritt der Nagellackbasis zuzufügen, um deren Schädigung durch gegebenenfalls einwirkende Scherkräfte weitgehend zu vermeiden.

Erfindungsgemäß wird wenigstens ein mikronisiertes Wachs in einem Nagellack verwendet.

Schließlich wird der der Erfindung zugrundeliegende Aufgabe durch Bereitstellung eines mit dem erfindungsgemäßen Nagellack versehenen Gegenstandes gemäß Anspruch 6 gelöst. Bei dem Gegenstand handelt es sich um einen künstlichen Fingernagel.

Unter einem Fingernagel wird im Sinne dieser Erfindung sowohl ein menschlicher Finger- bzw. Fußnagel als auch ein künstlicher Finger- bzw. Fußnagel verstanden.

Die Erfinder haben überraschend festgestellt, dass bei Verwendung von wenigstens einem mikronisiertem Wachs in einem farbmittelhaltigen Nagellack zugleich zwei Effekte erhalten werden. Zum einen weist der erfindungsgemäße Nagellack nach Applikation und Trocknung eine angenehme, samtige Haptik auf. Dies ist sehr erwünscht, wenn beispielsweise die lackierten Finger- und/ oder Fußnägel mit anderen Hautpartien in Kontakt kommen. Zum anderen bewirkt der Zusatz wenigstens eines mikronisierten Wachses, dass der erfindungsgemäße Nagellack nach Applikation und Trocknung einem hiermit lackierten (künstlichen) Fingernagel eine mattes, aber nicht stumpfes Erscheinungsbild verleiht. Weiterhin hat sich äußerst überraschend herausgestellt, dass der optische Sinneseindruck des erfindungsgemäßen Nagellacks in Gegenwart wenigstens eines Effektpigments nach Applikation und Trocknung weniger vom Betrachtungswinkel abhängig ist. Erfindungsgemäß enthält der Nagellack wenigstens ein plättchenförmiges Effektpigment.

Der erfindungsgemäße Nagellack kann wenigstens ein Farbmittel umfassen, welches sowohl ein Farbstoff als auch ein Pigment sein kann. Farbstoffe sind im umgebenden Medium lösliche, schwarze oder bunte Substanzen. Pigmente sind sphärische oder plättchenförmige Farbmittel, die im Gegensatz zu Farbstoffen im umgebenden Medium unlöslich sind (vgl. dazu DIN 55943: 2001-10 oder Römpp Lexikon Lacke und Druckfarben, Georg Thieme Verlag 1998, Stichworte Farbmittel, Farbstoffe, Pigmente). Zu den Pigmenten zählen sowohl plättchenförmige Effektpigmente, wie Metalleffekt- oder Perlglanzpigmente, als auch organische oder anorganische Pigmente.

Die im erfindungsgemäßen Nagellack bevorzugt einsetzbaren Farbmittel können unter anderem aus der entsprechenden Positivliste der Kosmetikverordnung (Verordnung (EG) Nr. 1223/2009, Anhang IV bzw. Code of Federal Regulations Title 21, part 73) ausgewählt werden.

Die in dem erfindungsgemäßen Nagellack einsetzbaren Metalleffektpigmente können entweder durch konventionelle Nass- oder Trockenvermahlung oder durch PVD-Verfahren hergestellt werden.

Geeignete metallische, gegebenenfalls zu beschichtende, plättchenförmige Substrate können aus der Gruppe, bestehend aus Aluminium-, Kupfer-, Zink-, Eisen-, Titan-, Edelstahl-, Gold-, Silberplättchen, deren Legierungen und deren Gemische, ausgewählt werden. Bevorzugt werden die metallischen plättchenförmigen Substrate aus der Gruppe, bestehend aus Aluminium-, Kupfer-, Zink-, Eisen-, Edelstahlplättchen, deren Legierungen und deren Gemische, ausgewählt. Besonders bevorzugt werden die metallischen plättchenförmigen Substrate aus der Gruppe, bestehend aus Aluminium-, Kupfer-, Zink-, Eisenplättchen, deren Legierungen und deren Gemische, ausgewählt. Ganz besonders bevorzugt werden die metallischen Substrate aus der Gruppe, bestehend aus Aluminium-, Kupfer-, Eisenplättchen, deren Legierungen und deren Gemische, ausgewählt. Insbesondere bevorzugt werden als metallische plättchenförmige Substrate Aluminiumplättchen, Kupferplättchen, deren Legierungen oder deren Gemische eingesetzt.

Speziell für den Einsatz in Nagellacken geeignete Metalleffektpigmente sind z.B. kommerziell erhältliche Aluminiumpigmentdispersionen wie Silverdream Moonlight 50 IL, Silverdream Starlight 70 IL, Metalure L-55350 AE oder Metalure Prismatic H-50550 AE (Fa. Eckart).

Sind die metallischen plättchenförmigen Substrate mit wenigstens einer Nachbeschichtung versehen, kann diese beispielsweise aus Metalloxiden und/ oder Metallhydroxiden von Titan, Aluminium, Eisen, Cer, Chrom, Silizium und/ oder Mischungen hiervon ausgewählt sein. Mit Metalloxiden beschichtete Metalleffektpigmente werden beispielsweise in EP 1 532 213 B1 sowie in EP 1 758 550 A2 beschrieben. Mit Siliziumdioxid beschichtete Metalleffektpigmente sind kommerziell, in Abhängigkeit von dem als Substrat verwendeten plättchenförmigen Metall bzw. verwendeter plättchenförmiger Metalllegierung, in den Farbtönen Silber, Goldbronze und Kupfer unter dem Handelnamen Visionaire (Fa. Eckart) erhältlich.

Dieses Farbspektrum lässt sich durch eingefärbte Metalleffektpigmente erweitern. Werden Aluminiumplättchen nasschemisch oxidiert, wie beispielsweise aus EP 0 848 735 B1 bekannt, können warme Champagnerfarbtöne erzielt werden. Die weitere Beschichtung nasschemisch oxidierter Aluminiumplättchen mit beispielsweise Eisenoxiden ermöglicht gemäß EP 1 685 198 B1 intensive Rotfarbtöne. Sind die Aluminiumeffektpigmente mit einer Farbpigmente enthaltenden Metalloxidschicht versehen, beeinflussen die Farbpigmente das Erscheinungsbild des resultierenden Aluminiumeffektpigments. Rot- bzw. Blaufarbtöne können beispielsweise durch die Verwendung von D & C Red 7 (CI 15 850), D & C Red 34 (CI 15 880), Carmine (CI 75 470) bzw. Berliner Blau (CI 77 400) als Farbpigment erreicht werden. Kommerziell erhältlich sind gefärbte Aluminiumeffektpigmente beispielsweise unter dem Handelsnamen Visionaire (Fa. Eckart).

Auch können magnetische Metalleffektpigmente, wie beispielsweise plättchenförmige Eisenpigmente, die mit einer Inhibitor- und/ oder Korrosionsschutzschicht stabilisiert sein können, in dem erfindungsgemäßen Nagellack eingesetzt werden. Derartige Eisenpigmente werden beispielsweise in der EP 1 251 152 B1 beschrieben und sind kommerziell beispielsweise unter der Bezeichnung Stapa WM Iron VP 401040 erhältlich (Fa. Eckart).

Für kosmetische Anwendungen sollten die in den Metalleffektpigmenten vorliegenden Verunreinigungen an Schwermetallen wie z.B. Quecksilber, Arsen, Blei, Cadmium möglichst gering sein, vorzugsweise < 70 ppm, bezogen auf das Gesamtgewicht des Metalleffektpigments. Vorzugsweise sollte der Quecksilbergehalt bei $\leq$ 1 ppm, der Arsengehalt bei $\leq$ 5 ppm, der Bleigehalt bei $\leq$ 40 ppm und der Cadmiumgehalt bei $\leq$ 15 ppm liegen, jeweils bezogen auf das Gesamtgewicht des Metalleffektpigments.

Alternativ bzw. zusätzlich zu den Metalleffektpigmenten können dem erfindungsgemäßen Nagellack Perlglanzpigmente basierend auf nichtmetallischen plättchenförmigen Substraten zugesetzt werden.

Die nichtmetallischen, gegebenenfalls zu beschichtenden, plättchenförmigen Substrate sind vorzugsweise im Wesentlichen transparent, bevorzugt transparent, d.h. für sichtbares Licht sind sie zumindest teilweise durchlässig. Die nichtmetallischen plättchenförmigen Substrate können aus der Gruppe, bestehend aus natürlichen Glimmerplätt-chen, synthetischen Glimmerplättchen, Glasplättchen, $SiO_2$-Plättchen, $Al_2O_3$-Plättchen, BiOCl-Plättchen, $TiO_2$-Plätt-chen, $Fe_2O_3$-Plättchen, Sericitplättchen, Kaolinplättchen, Graphitplättchen, Talkumplättchen, Polymerplättchen, plättchenförmigen Substraten, die eine anorganisch-organische Mischschicht umfassen, ausgewählt werden. Des Weiteren können als nichtmetallische plättchenförmige Substrate Glasplättchen, die beidseitig mit semitransparenten Metallschichten, ausgewählt aus der Gruppe, bestehend aus Silber, Aluminium, Chrom, Nickel, Gold, Platin, Palladium, Kupfer, Zink, Titan, deren Legierungen und Mischungen hiervon, beschichtet sind, verwendet werden. Erfindungsgemäß können als Perlglanzpigmente auch solche verwendet werden, deren Substrate Gemische der vorstehend angegebenen plättchenförmigen Substrate sind.

Bevorzugt werden die nichtmetallischen plättchenförmigen Substrate aus der Gruppe, bestehend aus natürlichen Glimmerplättchen, synthetischen Glimmerplättchen, Glasplättchen, $SiO_2$-Plättchen, $Al_2O_3$-Plättchen und deren Gemische, ausgewählt. Besonders bevorzugt werden die nichtmetallischen plättchenförmigen Substrate aus der Gruppe, bestehend aus natürlichen Glimmerplättchen, synthetischen Glimmerplättchen, Glasplättchen und deren Gemische, ausgewählt. Ganz besonders bevorzugt sind als Substrate natürliche und synthetische Glimmerplättchen und deren Gemische. Insbesondere ist als Substrat plättchenförmiger natürlicher Glimmer bevorzugt.

Natürliche Glimmerplättchen besitzen im Unterschied zu synthetischen plättchenförmigen transparenten Substraten den Nachteil, dass Verunreinigungen durch eingelagerte Fremddionen den Farbton verändern können, und dass die Oberfläche herstellungsbedingt nicht ideal glatt ist, sondern Unregelmäßigkeiten, wie z.B. Stufen aufweisen kann.

Synthetische Substrate wie beispielsweise Glasplättchen oder synthetischer Glimmer können dagegen glatte Oberflächen sowie eine einheitliche Dicke innerhalb eines einzelnen Substratteilchens sowie vorzugsweise über die Gesamtheit aller Substratteilchen aufweisen. Somit bietet die Oberfläche nur wenig Streuzentren für einfallendes bzw. reflektiertes Licht und ermöglicht so nach Beschichtung dieser plättchenförmigen Substrate höher glänzende und farbstärkere Perlglanzpigmente als mit plättchenförmigem natürlichem Glimmer als Substrat. Als Glasplättchen werden bevorzugt jene verwendet, die nach den in EP 0 289 240 A1, WO 2004/056716 A1 und der WO 2005/063637 A1 beschriebenen Verfahren hergestellt werden. Die als Substrat verwendbaren Glasplättchen können beispielsweise eine Zusammensetzung entsprechend der Lehre der EP 1 980 594 B1 aufweisen.

Der vorstehend beschriebene Unterschied zwischen auf plättchenförmigen transparenten natürlichen und synthetischen Substraten basierenden Perlglanzpigmenten ist auch in Nagellackapplikationen erkennbar. Üblicherweise verleihen auf synthetischen Substraten basierende Perlglanzpigmente einer Nagellackapplikation ein farbreineres und glänzenderes Aussehen.

Das nichtmetallische plättchenförmige Substrat, wie vorstehend beschrieben, kann mit wenigstens einer Schicht oder Beschichtung versehen werden, wobei die wenigstens eine Schicht vorzugsweise Metalloxide, Metalloxidhydrate, Metallhydroxide, Metallsuboxide, Metalle, Metallfluoride, Metalloxyhalogenide, Metallchalcogenide, Metallnitride, Metalloxynitride, Metallsulfide, Metallcarbide oder Mischungen davon umfasst. Gemäß einer bevorzugten Variante besteht die wenigstens eine Schicht oder Beschichtung aus den vorstehend genannten Materialien.

Bevorzugt werden als Schicht oder Beschichtung Metalloxide, Metalloxidhydrate, Metallhydroxide und/ oder deren Mischungen eingesetzt. Besonders bevorzugt werden Metalloxide und Metalloxidhydrate und/ oder deren Mischungen eingesetzt. Vorstehend genannte Materialien können entweder als separat voneinander getrennte Schichten oder auch nebeneinander in derselben Schicht vorliegen.

Die Begriffe Schicht oder Beschichtung werden austauschbar verwendet, sofern nicht anders angegeben.

Die Beschichtung kann entweder das Substrat vollständig umhüllen, nur partiell auf dem Substrat vorliegen oder nur dessen obere und/ oder untere Fläche bedecken.

Die wenigstens eine Schicht kann eine optisch wirksame Schicht sein und/ oder als Schutzschicht dienen.

Wird eine hochbrechende Schicht auf ein nichtmetallisches plättchenförmiges Substrat aufgebracht, so liegt der Brechungsindex bei $n \geq 1,8$, bevorzugt bei $n \geq 1,9$ und besonders bevorzugt bei $n \geq 2,0$. Im Falle einer niedrigbrechenden Schicht oder Beschichtung liegt der Brechungsindex bei $n < 1,8$, bevorzugt bei $n < 1,7$ und besonders bevorzugt bei $n < 1,6$.

Als hochbrechende Schicht eignen sich beispielsweise Metalloxide wie Titanoxid, vorzugsweise Titandioxid ($TiO_2$), Eisenoxid, vorzugsweise Eisen(III)oxid ($Fe_2O_3$) und/ oder Eisen(II/III)oxid ($Fe_3O_4$), Zinkoxid, vorzugsweise ZnO, Zinnoxid, vorzugsweise Zinndioxid ($SnO_2$), Zirkoniumoxid, vorzugsweise Zirkoniumdioxid ($ZrO_2$) Antimonoxid vorzugs-weise Antimon(III)oxid ($Sb_2O_3$), Magnesiumoxid, vorzugsweise MgO, Ceroxid, vorzugsweise Cer(IV)oxid ($CeO_2$) Co-baltoxid, vorzugsweise Cobalt(II)oxid (CoO) und/oder Cobalt(II/III)oxid ($Co_3O_4$), Chromoxid, vorzugsweise Chrom(III)oxid ($Cr_2O_3$), Kupferoxid, vorzugsweise Kupfer(I)oxid ($Cu_2O$) und/ oder Kupfer(II)oxid (CuO), Vanadiumoxid, vorzugsweise Vanadium(IV)oxid ($VO_2$) und/ oder Vanadium(III)oxid ($V_2O_3$), Metallsulfide wie Zinksulfid, vorzugsweise Zink(II)sulfid (ZnS), Metalloxidhydrate wie Goethit (FeOOH), Titanate wie Calciumtitanat ($CaTiO_3$) oder Eisentitanate, wie z.B. Ilmenit ($FeTiO_3$), Pseudobrookit ($Fe_2TiO_5$) und/ oder Pseudorutil ($Fe_2Ti_3O_9$), Metalle, wie z.B. Molybdän, Eisen, Wolfram, Chrom, Cobalt, Nickel, Silber, Palladium, Platin, deren Gemische und/ oder Legierungen, dotierte Metalloxide, wie beispielsweise Titandioxid und Zirkoniumdioxid, die mit selektiv absorbierenden Farbmitteln eingefärbt sind, und/ oder deren Gemische. Die zuletzt genannte Einfärbung nicht absorbierender hochbrechender Metalloxide kann z.B. durch Einbau von Farbmitteln in die Metalloxidschicht, durch deren Dotierung mit selektiv absorbierenden Metallkationen oder farbigen Metalloxiden wie Eisen(III)oxid oder durch Überziehen der Metalloxidschicht mit einem ein Farbmittel enthaltenden Film erfolgen.

Bevorzugt umfasst die hochbrechende Schicht Metalloxide, Metallhydroxide und/ oder Metalloxidhydrate. Besonders bevorzugt werden Metalloxide eingesetzt. Ganz besonders bevorzugt werden Titandioxid und/ oder Eisenoxid sowie Mischungen davon verwendet.

Wird mit Titandioxid beschichtet, kann das Titandioxid in der Rutil- oder Anataskristallmodifikation vorliegen. Die Rutilform kann beispielsweise erhalten werden, indem beispielsweise vor Aufbringung der Titandioxidschicht eine Schicht aus Zinndioxid auf das zu beschichtende plättchenförmige Substrat aufgebracht wird. Auf dieser Schicht aus Zinndioxid kristallisiert Titandioxid in der Rutilmodifikation. Das Zinndioxid kann dabei als separate Schicht vorliegen, wobei die Schichtdicke wenige Nanometer, beispielsweise weniger als 10 nm, weiter bevorzugt weniger als 5 nm, noch weiter bevorzugt weniger als 3 nm, betragen kann. Das Zinndioxid kann aber auch mit dem Titandioxid zumindest teilweise in Mischung vorliegen.

Beispiele für niedrigbrechende Schichten sind unter anderem Metalloxide wie Siliziumoxid, vorzugsweise Siliziumdioxid ($SiO_2$), Aluminiumoxid, vorzugsweise $Al_2O_3$, Boroxid, vorzugsweise Bor(III)oxid ($B_2O_3$), Metallfluoride wie Magnesiumfluorid, vorzugsweise $MgF_2$, Aluminiumfluorid, vorzugsweise $AlF_3$, Cerfluorid, vorzugsweise Cer(III)fluorid ($CeF_3$), Calciumfluorid, vorzugsweise $CaF_2$, Metalloxidhydrate wie Aluminiumoxidhydrat AlOOH, Siliziumoxidhydrat, vorzugsweise $SiO_2 \cdot H_2O$ und/ oder deren Gemische.

Vorzugsweise umfasst die niedrigbrechende Schicht Siliziumdioxid.

Ist das nichtmetallische plättchenförmige Substrat mit nur einer einzigen Metalloxidschicht beschichtet, so weist diese vorzugsweise einen hohen Brechungsindex auf. In Abhängigkeit von der geometrischen Metalloxidschichtdicke können derartige Perlglanzpigmente unterschiedliche Farbeffekte bewirken, wie in Tabelle 1 veranschaulicht.

Tabelle 1: Typische Farben und geometrische Schichtdicken von Perlglanzpigmenten

| | Belegung/ geometrische Schichtdicke | Farbe |
|---|---|---|
| Silberweiße Perlglanzpigmente | $TiO_2$: 40 - 60 nm | silber |
| Interferenzpigmente | $TiO_2$: 20-40 nm | fahlblau |
| | $TiO_2$: 60 - 80 nm | gelb |
| | $TiO_2$: 80-100nm | rot |
| | $TiO_2$: 100-140 nm | blau |
| | $TiO_2$: 120-160nm | grün |
| | $TiO_2$: 280 - 320 nm | grün (III. Ordnung) |
| Farbglanzpigmente | $Fe_2O_3$: 35 - 45 nm | bronze |
| | $Fe_2O_3$: 45 - 55 nm | kupfer |
| | $Fe_2O_3$: 55 - 65 nm | rot |
| | $Fe_2O_3$: 65 - 75 nm | rotviolett |
| | $Fe_2O_3$: 75 - 85 nm | rotgrün |
| | $Fe_3O_4$ | schwarz |
| Kombinationspigmente | $TiO_2$/ $Fe_2O_3$ | Goldtöne |
| | $TiO_2$/ $Cr_2O_3$ | grün |
| | $TiO_2$/ Berliner Blau | tiefblau |

Mit $TiO_2$ und/ oder Eisenoxid beschichtete Perlglanzpigmente auf Basis von plättchenförmigem natürlichem Glimmer sind beispielsweise unter dem Namen Prestige im Handel erhältlich (Fa. Eckart). Besteht das plättchenförmige transparente Substrat aus synthetischem Glimmer sind derartige Perlglanzpigmente z.B. unter dem Handelsnamen SynCrystal kommerziell erhältlich (Fa. Eckart). Mit $TiO_2$ und/ oder Eisenoxid beschichtete $Al_2O_3$-Flakes und entsprechend beschichtete $SiO_2$-Flakes werden beispielsweise unter dem Handelsnamen Xirona angeboten (Fa. Merck). Mit $TiO_2$ und/ oder Eisenoxid beschichtete Glasplättchen werden z.B. unter dem Namen Mirage (Fa. Eckart), unter der Bezeichnung Reflecks (Fa. BASF Catalysts) oder unter der Bezeichnung Ronstar (Fa. Merck) angeboten.

Perlglanzpigmente, basierend auf künstlichen Substraten und charakterisiert durch die Kennzahlen $D_{10}$, $D_{50}$ und $D_{90}$ aus der Summenhäufigkeitsverteilung der volumengemittelten Größenverteilungsfunktion mit einem Span $\Delta D$ = $(D_{90} - D_{10})/ D_{10}$ von 0,7 bis 1,4 zeichnen sich gemäß WO 2009/103322 A1 durch ihre hohe Farbreinheit aus.

Die nichtmetallischen plättchenförmigen Substrate können auch mit einem mehrlagigen Schichtaufbau mit oder bestehend aus Metalloxid, Metallhydroxid, Metallsuboxid und/ oder Metalloxidhydrat beschichtet sein, wobei die Reihenfolge der Schichten variabel sein kann. Bevorzugt ist hierbei eine Schichtenfolge, bei dem wenigstens eine hochbrechende Schicht und wenigstens eine niedrigbrechende Schicht in alternierender Weise auf einem Substrat angeordnet sind. Bei der alternierenden Anordnung ist es auch möglich, ein oder mehrere hochbrechende Schichten unmittelbar übereinander und nachfolgend ein oder mehrere niedrigbrechende Schichten unmittelbar übereinander anzuordnen. Wesentlich ist jedoch, dass in dem Schichtaufbau hoch- und niedrigbrechende Schichten vorkommen. Vorzugsweise sind aufgehend vom plättchenförmigen Substrat wenigstens eine hoch-, niedrig- und wiederum hochbrechende Schicht angeordnet, was in Perlglanzpigmenten mit besonders intensiven Interferenzfarben resultiert. Die Interferenzfarbe kann hierbei in Abhängigkeit von Schichtaufbau und -dicken silbern oder nicht silbern sein.

Außerdem sind auch Pigmente mit Interferenzeffekt, die kein Substrat aufweisen, z.B. Flüssigkristalle wie Helicone (Fa. LCP Technology) oder Partikel mit opaleszierendem Effekt, bestehend aus monodispersen Kugeln in einer dreidimensionalen, domänenweise dicht gepackten und regelmäßig angeordneten Struktur, beispielsweise beschrieben in der WO 2001/88044 A1, einsetzbar. Darüber hinaus können auch holografische Glitterpigmente, wie z.B. Geometrie Pigments (Fa. Spectratek Tech), Fluoreszenzpigmente, Phosphoreszenzpigmente, photochrome Pigmente, thermochrome Pigmente und sogenannte "Quantum Dots" (Fa. Quantum Dots Corporation), im erfindungsgemäßen Nagellack Verwendung finden.

Ferner können auch "Glitter'-Effektpigmente, welche beispielsweise unter der Handelsbezeichnung Metasomes Standard/Glitter in verschiedenen Farben (gelb, rot, grün, blau; Fa. Floratech) erhältlich sind, eingesetzt werden. Die Glitterpartikel können hierbei in Gemischen mit verschiedenen Hilfs- und Farbmitteln, wie beispielsweise den Farbmitteln mit den Colour Index (CI) Nummern 19 140, 77 007, 77 289, 77 491, vorliegen.

Als Farbmittel können beispielsweise eine oder mehrere Substanzen aus der folgenden Gruppe im erfindungsgemäßen Nagellack Verwendung finden: 2,4-Dihydroxyazobenzol, 1-(2'-Chlor-4'-nitro-1'-phenylazo)-2-hydroxynaphthalin, Ceresrot, 2-(4-Sulfo-1-naphthylazo)-1-naphthol-4-sulfonsäure, Calciumsalz der 2-Hydroxy-1,2'-azonaphthalin-1'-sulfonsäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfonsäure, Aluminiumsalz der 1-(4-Sulfo-1-naphthylazo)-2-naphthol-3,6-disulfonsäure, 1-(4-Sulfo-1-naphthylazo)-2-naphthol-6,8-disulfonsäure, Aluminium- und Zirkoniumsalze von 4,5-Dibrom-fluorescein, Aluminium- und Zirkoniumsalze von 2,4,5,7-Tetrabromfluorescein, Aluminiumsalz von 3',4',5',6'-Tetrachlor-2,4,5,7-tetrabromfluorescein, Aluminiumsalz von 2,4,5,7-Tetraiodfluorescein, Aluminiumsalz der Chinophthalondisulfonsäure, Aluminiumsalz der Indigodisulfonsäure. Ferner einsetzbar sind Naturfarbstoffe, wie z. B. Paprikaextrakt, ß-Carotin oder Cochenille.

Geeignete organische Pigmente für den Einsatz in den erfindungsgemäßen Nagellacken umfassen beispielsweise Nitroso-, Nitro-, Azo-, Xanthen-, Chinolin-, Anthrachinon-, Phthalocyanin-, Metallkomplex-, Isoindolinon-, Isoindolin-, Chinacridon-, Perinon-, Perylen-, Diketopyrrolopyrrol-, Thioindigo-, Dioxazin-, Triphenylmethan- und Chinophthalonverbindungen. Des Weiteren können die organischen Pigmente beispielsweise ausgewählt sein aus Carmine, Carbon Black, Anilinschwarz, Chinacridon, Phthalocyaninblau, D & C Red 21 (CI 45 380), D & C Orange 5 (CI 45 370), D & C Red 27 (CI 45 410), D & C Orange 10 (CI 45 425), D & C Red 3 (CI 45 430), D & C Red 7 (CI 15 850), D & C Red 4 (CI 15 510), D & C Red 33 (CI 17 200), D & C Red 34 (CI 15 880) D & C Yellow 5 (CI 19 140), D & C Yellow 6 (CI 15 985), D & C Green (CI 61 570), D & C Yellow 10 (CI 77 002), D & C Green 3 (CI 42 053) und / oder D & C Blue 1 (CI 42 090).

Geeignete anorganische Pigmente umfassen beispielsweise Metalloxide und/ oder andere in Wasser schwerlösliche oder unlösliche Metallverbindungen, insbesondere Metalloxide von Titan, beispielsweise Titandioxid (CI 77891), Zink, Eisen, beispielsweise rotes und schwarzes Eisenoxid (CI 77491 (rot), 77499 (schwarz)) oder Eisenoxidhydrat (CI 77492, gelb), Zirkonium, Silizium, Mangan, Aluminium, Cer, Chrom sowie Mischoxide der genannten Metalle und deren Mischungen. Weitere geeignete anorganische Pigmente sind beispielsweise Bariumsulfat-, Zinksulfid-, Manganviolett-, Ultramarinblau- und/ oder Berliner Blau-Pigmente.

Die organischen und anorganischen Farbpigmente werden in der Regel in einer Perlmühle oder Mahlkörpermühle, z.B. mit Zirkonoxidkugeln, angerieben. Die Anreibung kann in organischem Lösemittel, beispielsweise in Testbenzin oder Isopropanol, erfolgen. Hinsichtlich der Kompatibilität mit handelsüblichen Nagellacksystemen ist Isopropanol bevorzugt.

Die Pigmente können oberflächenmodifiziert sein, wobei beispielsweise ein hydrophiler, amphiphiler oder hydrophober Charakter gebildet wird bzw. erhalten bleiben soll. Diese Oberflächenbehandlung kann darin bestehen, dass die Pigmente nach für den Fachmann bekannten Verfahren mit einer dünnen hydrophilen und/ oder hydrophoben anorganischen und/ oder organischen Schicht versehen werden.

Der erfindungsgemäße Nagellack umfasst neben wenigstens einem Farbmittel wenigstens ein mikronisiertes Wachs. Mikronisierte Wachse oder Mikrowachse sind lipophile Verbindungen mit einer mittleren Teilchengröße $D_{50}$ aus einem Bereich von 2,5 bis 91 $\mu$m, bevorzugt aus einem Bereich von 3 bis 79 $\mu$m, noch weiter bevorzugt aus einem Bereich von 4 bis 69 $\mu$m, besonders bevorzugt aus einem Bereich von 5 bis 59 $\mu$m und ganz besonders bevorzugt aus einem Bereich von 6 bis 49 $\mu$m. Die mikronisierten Wachse liegen vorzugsweise als diskrete

Wachseinzelpartikel vor, es kann sich aber auch um Aggregate mit identischer mittlerer Teilchengröße $D_{50}$ handeln.

Darüber hinaus weist das mikronisierte Wachs bevorzugt nur wenige, vorzugsweise keine, Teilchen im Größenbereich > 200 $\mu$m auf, da sonst der beschriebene optische und haptische Effekt im applizierten und getrockneten Nagellack nicht mehr erhalten werden kann. Das menschliche Auge kann Partikel mit einer Größe von > 200 $\mu$m erkennen, so dass von einem Betrachter des applizierten und getrockneten Nagellacks kein homogenes Erscheinungsbild erkannt wird. Im widrigsten Fall werden solche groben Wachspartikel als Fehllackierung, d.h. als unerwünschte Erhöhungen im Lackfilm, wahrgenommen. Auch haptisch werden Wachspartikel mit einem Partikeldurchmesser von > 200 $\mu$m als unangenehm, bisweilen sogar kratzend empfunden.

Der $D_{10}$-, $D_{50}$- bzw. $D_{90}$-Wert der Summenhäufigkeitsverteilung der volumengemittelten Größenverteilungsfunktion, wie sie durch Laserbeugungsmethoden erhalten wird, gibt an, dass 10%, 50% bzw. 90% der mikronisierten Wachse bzw. der Pigmente einen Durchmesser aufweisen, der gleich oder kleiner als der jeweils angegebene Wert ist. Hierbei wird die Größenverteilungskurve der mikronisierten Wachse und Perlglanzpigmente mit einem Gerät der Fa. Malvern (Gerät: MALVERN Mastersizer 2000), die Größenverteilungskurve der Metalleffektpigmente (in Pulverform) mit einem Gerät der Fa. Sympathec (Gerät: HELOS Partikelgrößenanalyse mit RODOS Trockendispergierung und Dosieraggregat VIBRI) und die Größenverteilungskurve der Metalleffektpigmente (in Pastenform), der organischen und anorganischen Pigmente mit einem Gerät der Fa. Quantachrome (Gerät: Cilas 1064), jeweils gemäß Herstellerangaben bestimmt. Die Auswertung der Streulichtsignale erfolgte dabei nach der Fraunhofer-Methode bzw. Mie-Theorie, welche auch Brechungs- und Absorptionsverhalten der mikronisierten Wachse bzw. Pigmente beinhaltet.

Mikronisierte Wachse sind üblicherweise bei Raumtemperatur (25°C) fest und gehen oberhalb ihres Schmelzbereichs ohne Zersetzung vom festen in den flüssigen Zustand über. Die Konsistenz der mikronisierten Wachse kann von fest bis brüchig hart reichen. Mikronisierte Wachse können durchscheinend bis opak sein, sie können weiterhin zusätzlich mit einem kosmetisch zulässigen Farbmittel eingefärbt werden. Die mikronisierten Wachse können bereits bei einer Temperatur von 40°C zu schmelzen beginnen. Bevorzugt liegt der Schmelzbereich zwischen 51 und 399°C, weiter bevorzugt zwischen 71 und 389°C, besonders bevorzugt zwischen 91 und 379°C und insbesondere bevorzugt zwischen 111 und 369°C. Der Schmelzbereich der mikronisierten Wachse wird hierbei gemäß der DGF (Deutsche Gesellschaft für Fettwissenschaften) Einheitsmethode C-IV 3a mit einem Schmelzpunktmessgerät (Modell 5A 6797, Fa. Gallenkamp) bestimmt.

Als mikronisierte Wachse können natürliche und/ oder synthetische Wachse eingesetzt werden. Weiterhin können als mikronisierte Wachse vorzugsweise mit kosmetisch zugelassenen Farbmitteln eingefärbte natürliche und/ oder synthetische Wachse verwendet werden. Außerdem können auch Mischungen von jeweils unterschiedlichen gefärbten und/ oder ungefärbten mikronisierten Wachsen dem erfindungsgemäßen Nagellack zugegeben werden.

Als natürliche mikronisierte Wachse können beispielsweise mikronisiertes Bienenwachs (INCI: Cera Alba Beeswax), mikronisiertes Candellilawachs (INCI: Candellila Cera) oder mikronisiertes Carnaubawachs (INCI: Copernicia Cerifera (Carnauba) Wax), kommerziell erhältlich z.B. als Carnauba Super-Micropowder (Fa. Kahl) oder Microcare 350 (Fa. Micropowders) eingesetzt werden. Es können selbstverständlich auch verschiedene natürliche mikronisierte Wachse dem erfindungsgemäßen Nagellack zugesetzt werden.

Ebenfalls zum Einsatz kommen können Gemische natürlicher und synthetischer mikronisierter Wachse. Hierzu zählen u.a. mikronisiertes Carnauba-/ PE-Wachs (INCI: Carnauba/ Polyethylene), kommerziell erhältlich als z.B. Microcare 300, Microcare 310 (Fa. Micro Powders) oder mikronisiertes Carnauba-/ synthetisches Wachs (INCI: Carnauba/ Synthetic Wax), kommerziell erhältlich als z.B. Microcare 325 (Fa. Micro Powders).

Als mikronisierte Wachse können u.a. synthetische Wachse (INCI: (Oxidized) Synthetic Wax), kommerziell erhältlich z.B. als Microease 110XF, Microease 110S, Microease 114 S, Microease 116 (Fa. Micro Powders), mikronisierte PE-Wachse, kommerziell erhältlich z.B. als Ceraflour 990 oder Ceraflour 991 (Fa. Byk-Chemie), als PE Super Micropowder (INCI: Polyethylene, Fa. Kahl), als Micropoly 200, Micropoly 210, Micropoly 220, Micropoly 220L, Micropoly 250S (INCI: (Oxidized) Polyethylene, Fa. Micro Powders) oder als PE Microspheres (Fa. Cospheric), mikronisierte PP-Wachse, kommerziell erhältlich als Ceraflour 913, Ceraflour 914, Ceraflour 915, Ceraflour 916 (Fa. Byk-Chemie) oder als Mattewax 511 (INCI: Polypropylene, Fa. Micro Powders), mikronisierte PMMA Wachse (Polymethylmethacrylat, CAS: 9011-14-7), kommerziell erhältlich z.B. als PMMA Microspheres (Fa. Cospheric) oder mikroniersierte Polytetrafluorethylenwachse (INCI: PTFE), kommerziell erhältlich als z.B. PTFE-Super Micropowder (Fa. Kahl) oder Microslip 519, Microslip 519 L (Fa. Micro Powders) eingesetzt werden.

Weiterhin können auch Gemische verschiedener mikronisierter synthetischer Wachse wie beispielsweise aus PE-/ Polytetrafluorethylen (INCI: Polyethylene/ PTFE), kommerziell erhältlich z.B. als PE-PTFE Super Micropowder der Fa. Kahl oder Microsilk 419 der Fa. Micro Powders, aus PE-/ Polytetrafluorethylen/ synthetischen Wachs (INCI: Polyethylene/ PTFE/ Synthetic Wax), kommerziell erhältlich z.B. als Microsilk 418 der Fa. Micro Powders, aus PP-/Polytetrafluorethylen (INCI: Polypropylene/ PTFE), kommerziell erhältlich u.a. als Microsilk 920 der Fa. Micro Powders Verwendung finden.

Auch mit Farbmitteln eingefärbte synthetische mikronisierte Wachse können dem erfindungsgemäßen Nagellack zugegeben werden. Zu erwähnen sind hier u.a. die gefärbten Micropheres, wie White, Black, Blue, Green, Orange, Pink, Purpie, Red, Yellow oder Grey Microspheres (Fa. Cospheric).

Je nach Nagellackbasis können die mikronisierten Wachse dem erfindungsgemäßen Nagellack gegebenenfalls auch als Dispersion zugegeben werden. Beispiele für kommerziell erhältliche Dispersionen (Fa. Micro Powders) sind Microspersion 220PC (INCI: Polyethylene, Isohexadecane), Microspersion 419PC (INCI: Polyethylene, PTFE, Isohexadecane), Microspersion 511 PC (INCI: Polypropylene, Isodecane, Polyethylene) oder Microspersion 519PC (INCI: PTFE, Isodecane, Polyethylene).

Vorzugsweise werden dem erfindungsgemäßen Nagellack ungefärbte synthetische mikronisierte Wachse zugegeben.

Bei einer weiteren Ausführungsform können auch Mischungen, die wenigstens ein mikronisiertes Wachs und wenigstens eine Sorte an hohlen Glaskügelchen enthalten, dem erfindungsgemäßen Nagellack zugegeben werden. Als hohle Glaskügelchen können beispielsweise Hollow Gläss Microspheres (Natriumsilikat, CAS Nr. 1344-09-8, Natriumborat CAS Nr. 7775-19-1, amorphes Siliziumdioxid CAS Nr. 7631-86-9; Fa. Cospheric) eingesetzt werden. Die mittlere Teilchengröße $D_{50}$ der hohlen Glaskügelchen liegt in einem Bereich von 27 bis 90 $\mu$m, bevorzugt in einem Bereich von 45 bis 75 $\mu$m und besonders bevorzugt in einem Bereich von 53 bis 63 $\mu$m. Gemäß einer bevorzugten Ausführungsform beträgt der Anteil an mikronisiertem Wachs, bezogen auf das Gesamtgewicht an mikronisiertem Wachs und hohlen Glaskügelchen, mehr als 50 Gew.-%, weiterhin bevorzugt mehr als 65 Gew.-%.

Bei einer bevorzugten Ausführungsform umfasst der erfindungsgemäße Nagellack neben dem wenigstens einen mikronisierten Wachs wenigstens ein Effektpigment, bevorzugt wenigstens ein Perlglanzpigment und/ oder wenigstens ein Metalleffektpigment.

Bei einer weiteren Ausführungsform kann dem erfindungsgemäßen Nagellack eine Mischung aus gefärbten und ungefärbten mikronisierten Wachsen gleicher oder verschiedener Teilchengröße und/ oder gleicher oder verschiedener chemischer Zusammensetzung der Wachsmatrix in Gegenwart wenigstens eines Effektpigments zugegeben werden.

Die Herstellung des erfindungsgemäßen Nagellacks kann durch Vermengen von wenigstens einem mikronisierten Wachs, wenigstens einem Farbmittel und einer Nagellackbasis erfolgen.

Die Zugabereihenfolge der für haptischen und/ oder optischen Effekt verantwortlichen Komponenten des erfindungsgemäßen Nagellacks ist nicht entscheidend. Das wenigstens eine mikronisierte Wachs kann dem efindungsgemäßen Nagellack in fester Form zugegeben werden. Das wenigstens eine mikronisierte Wachs wird dem erfindungsgemäßen Nagellack bevorzugt in fester Form zugegeben. Vorzugsweise werden die in der Nagellackbasis nicht löslichen Komponenten des erfindungsgemäßen Nagellacks, also mikronisierte(s) Wachs(e) und Pigment(e), nacheinander oder als Mischung einer mit einem löslichen Farbstoff eingefärbten oder nicht eingefärbten Nagellackbasis zugefügt. Die Herstellung des erfindungsgemäßen Nagellacks ist somit sehr einfach möglich. Um die Beschädigung plättchenförmiger Effektpigmente durch gegebenenfalls einwirkende Scherkräfte weitgehend zu vermeiden, ist es bevorzugt, diese gegen Ende des Vermengens oder Vermischens der Komponenten zuzugeben.

Mit Nagellackbasis ist erfindungsgemäß die Zusammensetzung gemeint, die die für einen Nagellack erforderlichen Komponenten wie Filmbildner, Harze, Weichmacher, Lösemittel und Thixotropiermittel enthält, jedoch noch nicht Farbmittel und mikronisierte(s) Wachs(e). Optional können der Nagellackbasis zusätzlich weitere Komponenten, z.B. Konservierungsmittel, UV-Absorber oder Duftstoffe zugeben werden.

Bei der Nagellackbasis kann es sich um eine kommerziell erhältliche Nagellackbasis wie beispielsweise International Lacquers Nailpolish & Care Base 359 (Fa. International Lacquers) oder um die Intermediates (Fa. Durlin) handeln. Weiterhin kann auch eine UV-härtende Nagellackbasis eingesetzt werden. Alternativ kann die Nagellackbasis nach den dem Fachmann bekannten Methoden unter Verwendung der gängigen Rohstoffe direkt formuliert werden. Als Filmbildner können beispielsweise Nitrocellulose, Ethylcellulose oder Celluloseacetat und als Harze z.B. Polyesterharze oder Polyvinylacetat eingesetzt werden. Als Weichmacher sind beispielsweise Dibutylphthalat, Tricresylphosphat, Triethylcitrat oder Campher zu nennen. Als gängige Lösemittel werden z.B. Ethylacetat, Isopropanol oder n-Butylacetat verwendet. Zu den Thixotropiermitteln zählen unter anderem Stearylalkoniumbentonit oder -hectorit.

Das wenigstens eine Farbmittel sowie das wenigstens eine mikronisierte Wachs ist mit einer Vielzahl an kommerziell erhältlichen gefärbten oder ungefärbten Nagellackbasissystemen kompatibel. Durch die unzähligen Kombinationsmöglichkeiten von wenigstens einem Farbmittel und wenigstens einem mikronisierten Wachs können auf sehr einfache Weise in Abhängigkeit von verwendetem Farbmittel und verwendetem Wachs unterschiedliche optische und haptische Effekte erzielt werden.

Um diese einzigartige Kombination aus optischem und haptischem Effekt zu erzielen, ist überraschenderweise keine mehrmalige Lackierung eines Fingernagels notwendig. Da in der Regel das den haptischen Effekt überwiegend hervorrufende mikronisierte Wachs dem Nagellack direkt in fester Form zugegeben wird, ist kein komplizierter mehrschichtiger Lackschichtaufbau auf einem Fingernagel mit beispielsweise einer für den haptischen und einer für den optischen Effekt verantwortlichen Schicht notwendig. Der erfindungsgemäße Nagellack weist nach Applikation und Trocknung überraschenderweise eine hervorragende mechanische Stabilität auf und wird durch gängige Haus- und/ oder Büroarbeit nicht beschädigt, sondern bleibt über einen Zeitraum von wenigstens zwei Tagen in optisch ansprechender Weise auf dem lackierten Fingernagel erhalten. Weiterhin zeigt der erfindungsgemäße Nagellack nach Applikation und Trocknung eine ausreichende chemische Stabilität gegenüber Hautpflegeprodukten, wie beispielsweise einer Handcreme und/ oder haushaltsüblichen Reinigungsmitteln, z.B. Spülmitteln.

Die unzähligen Kombinationsmöglichkeiten unterschiedlicher Farbmittel mit verschiedenen gefärbten oder ungefärbten mikronisierten Wachsen in jeweils unterschiedlicher Teilchengröße ermöglichen auf einfache Weise eine Vielzahl an optischen und haptischen Effekten. Ein Effektpigment in verschiedenen Teilchengrößen, wie z.B. ein Metalleffektpigment, kann mit einem mikronisierten Wachs in verschiedenen Teilchengrößen derart kombiniert werden, dass das Metalleffektpigment mit einer geringen Teilchengröße mit z.B. einer mittleren Teilchengröße $D_{50}$ aus einem Bereich von 5 bis 27 $\mu$m jeweils mit mikronisierten Wachsen einer geringen ($D_{50}$ aus einem Bereich von 2,5 bis 20 $\mu$m), einer mittleren ($D_{50}$ aus einem Bereich von > 20 bis 30 $\mu$m) und/ oder einer groben Teilchengröße ($D_{50}$ aus einem Bereich von > 30 bis 91 $\mu$m) im erfindungsgemäßen Nagellack vorliegt. Entsprechendes gilt selbstverständlich auch für Metalleffektpigmente mit mittleren ($D_{50}$ aus einem Bereich von > 27 bis 45 $\mu$m) oder groben ($D_{50}$ aus einem Bereich von > 45 bis 71 $\mu$m) Teilchengrößen. Neben einer Variation der mikronisierten Wachse kann das Metalleffektpigment in unterschiedlichen Teilchengrößen auch mit wenigstens einem weiteren Metalleffektpigment, einem Perlglanzpigment, einem organischen und/ oder anorganischen Farbpigment gleicher oder verschiedener Teilchengröße oder in Gegenwart eines im jeweiligen Nagellacksystem gelöst vorliegenden Farbmittels eingesetzt werden. Somit kann z.B. auch eine Mischung eines Aluminiumeffektpigments und eines Goldbronzeeffektpigments in Gegenwart eines mikronisierten Wachses vorliegen. Es können im erfindungsgemäßen Nagellack nicht nur die Farbmittel je nach gewünschter Farbe und zu erzielendem optischen Effekt variiert bzw. in verschiedener Weise untereinander kombiniert werden, sondern auch die eingesetzten mikronisierten Wachse können in unterschiedlichsten Teilchengrößen eingesetzt werden, so dass durch das Zusammenspiel beider Komponenten eine einzigartige Kombination optischer und haptischer Effekte verwirklicht werden kann. Ein erfindungsgemäßer Nagellack kann hierbei auch eine Mischung von mikronisierten Wachsen gleicher oder verschiedener Teilchengröße umfassen. Die vorstehend beispielhaft und nicht abschließend aufgeführten Kombinationsmöglichkeiten für Metalleffektpigmente gelten entsprechend auch für zu verwendende Perlglanzpigmente, organische oder anorganische Pigmente. Die Nagellackbasis kann hierbei ungefärbt oder mit wenigstens einem löslichen Farbstoff gefärbt vorliegen.

Eine Besonderheit bei der Verwendung magnetischer Effektpigmente, wie beispielsweise plättchenförmiger Eisenpigmente, ist, dass diese im erfindungsgemäßen Nagellack trotz der Anwesenheit des wenigstens einen mikronisierten Wachses magnetisch noch orientierbar sind. Die magnetischen Effektpigmente können auf einem frisch lackierten Fingernagel unter Anlegung eines äußeren Magnetfeldes unter Erzeugung sichtbarer Muster ausgerichtet und die in Abhängigkeit vom verwendeten Magnet erzeugten Muster durch Trocknen fixiert werden. Ein lackierter Fingernagel weist mithin zusätzlich zu den bereits beschriebenen optischen und haptischen Effekten durch das angelegte Magnetfeld induzierte Muster auf. Selbstverständlich können auch die magnetischen Effektpigmente in Abmischung mit weiteren Farbmitteln im erfindungsgemäßen Nagellack vorliegen. Wird eine Nagellackbasis, die gegebenenfalls mit einem Farbstoff eingefärbt sein kann, durch Zugabe wenigstens eines mikronisierten Wachses modifiziert, so ist die am lackierten Fingernagel im Vergleich zu der Nagellackbasis ohne Zugabe von mikronisiertem Wachs wahrnehmbare haptische Effektänderung ausschließlich auf das wenigstens eine mikronisierte Wachs zurückzuführen. Mit zunehmender Teilchengröße des wenigstens einen mikronisierten Wachses wird einem derart lackierten Fingernagel ein zunehmender samtartiger Effekt, der auch als "soft touch" Effekt bezeichnet werden kann, verliehen.

In Gegenwart eines organischen oder anorganischen Farbpigments beeinflusst das mikronisierte Wachs nach Applikation und Trocknung des erfindungsgemäßen Nagellacks in der Regel überwiegend den haptischen Eindruck. Sowohl bei den im Nagellack löslichen Farbmitteln als auch bei organischen oder anorganischen Absorptionspigmenten nimmt ein Betrachter über den kompletten Fingernagel winkelunabhängig im Wesentlichen den gleichen Farbeindruck wahr. Durch die Zugabe wenigstens eines mikronisierten Wachses verliert ein derart eingefärbter bzw. pigmentierter Nagellack seinen ursprünglichen Glanz und wirkt matter. Dieser Effekt kann in Abhängigkeit der Teilchengröße des wenigsten einen mikronisierten Wachses unterschiedlich stark ausgeprägt sein. Je größer die Teilchengröße des wenigstens einen mikronisierten Wachses ist, desto matter wirkt ein mit wenigstens einem Absorptionspigment versehener Nagellack. Auch die zugefügte Menge des wenigstens einen mikronisierten Wachses hat einen Einfluss auf die haptischen und optischen Eigenschaften des erfindungsgemäßen Nagellacks. Je höher der gewichtsmäßig prozentuale Anteil des wenigstens einen mikronisierten Wachses in der Nagellackrezeptur ist, desto matter erscheint der applizierte und getrocknete Nagellack einem Betrachter.

Im Unterschied zu Absorptionspigmenten zeigen plättchenförmige Effektpigmente ein winkelabhängiges Erscheinungsbild. Die optische Wirkung der plättchenförmige Effektpigmente beruht auf der gerichteten Reflexion von Licht an überwiegend flächig ausgebildeten, zueinander im Wesentlichen parallel ausgerichteten Pigmentteilchen. Je nach Substrat und darauf aufgebrachter(n) Beschichtung(en) erzeugen Interferenz-, Reflexions- und/ oder Absorptionsphänomene unterschiedliche Farb- und/ oder Helligkeitseindrücke. Ein menschlicher Fingernagel stellt durch seine schwache Krümmung und seine im Vergleich zur menschlichen Haut glattere Oberfläche einen guten Untergrund für eine Applikation von mit Effektpigmenten pigmentierten Nagellacken dar. Mithin ist auch an einem Fingernagel, welcher mit einem effektpigmentierten Nagellack lackiert wurde, der für das jeweilige Effektpigment typische winkelabhängige optische Eindruck deutlich erkennbar. Im Unterschied zu mit Absorptionspigmenten versetzten Nagellacken zeigen mit Effektpigmenten pigmentierte Nagellacke winkelabhängig ein unterschiedliches Erscheinungsbild. Ein Fingernagel, welcher mit einem Metalleffektpigmente enthaltenden Nagellack lackiert ist, zeigt im Glanzwinkel (45°) den typischen Metallglanz. Außerhalb des Glanzwinkels wirken derartige Nagellacke weniger glänzend und dunkler. Dieses Phänomen wird als Hell-/ Dunkelflop bezeichnet. Ist ein Fingernagel mit einem Perlglanzpigmente umfassenden Nagellack versehen, so verleiht dieser dem Fingernagel im Glanzwinkel den für Perlglanzpigmente typischen Tiefenglanz bei gleichzeitiger Transparenz. Auch dieser Effekt geht außerhalb des Glanzwinkels zunehmend verloren.

In Gegenwart wenigstens eines mikronisierten Wachses zeigen Nagellacke, die Effektpigmente enthalten, verglichen mit der entsprechender Nagellackapplikation ohne mikronisiertes Wachs, in der Applikation zwar einen Effekt- und/ oder Glanzverlust im Glanzwinkel. Allerdings zeigen derartige Applikationen aber überraschenderweise über den kompletten Winkelbereich für einen Betrachter einen einheitlicheren optischen Effekt, wobei es sich in Abhängigkeit von dem wenigstens einen eingesetzten Effektpigment hierbei um Glanz-, Helligkeits- und/ oder Interferenzeffekte handelt. Die für Effektpigmente charakteristische Winkelabhängigkeit des optischen Eindrucks wird somit gezielt abgeschwächt. Neben diesem optischen Effekt macht sich auch hier die Gegenwart wenigstens eines mikronisierten Wachses in haptischer Hinsicht bemerkbar. Bei Verwendung von Effektpigmenten ist der haptische Effekt nicht ausschließlich auf das mikronisierte Wachs zurückzuführen, sondern auch das wenigstens eine Effektpigment spielt hier je nach Teilchengröße und/ oder Pigmentierungshöhe eine gewisse Rolle. Ein Effektpigment sehr feiner Teilchengröße, also mit einer mittleren Teilchengröße $D_{50}$ aus einem Bereich von 1 bis 15 $\mu$m spielt in einem Vergleich von Nagellackapplikationen enthaltend ein mikronisiertes Wachs mit zunehmender Teilchengröße hinsichtlich des haptischen Effekts eine untergeordnete Rolle. Der optische Effekt eines derartigen Vergleichs ändert sich von einem sehr homogenen und seidig wirkenden zu einem mehr lebendig wirkenden Erscheinungsbild. Effektpigmente gewinnen mit zunehmender Teilchengröße mehr Einfluss auf den optischen und haptischen Effekt. Außerdem sind Effektpigmente mit größerer Teilchengröße, beispielsweise einer mittleren Teilchengröße $D_{50}$ aus einem Bereich von > 15 bis 100 $\mu$m, für glitzernde Nagellackapplikationen verantwortlich. Dieser Glitzereffekt ist umso stärker ausgeprägt je größer das im Nagellack vorliegende mikronisierte Wachs ist.

Neben den hier explizit beschriebenen optischen und haptischen Effekten wird schon aus der Vielzahl an Kombinationsmöglichkeiten von wenigstens einem Farbmittel und wenigstens einem mikronisierten Wachs ersichtlich, dass noch deutlich mehr unterschiedliche Effekte erzielt werden können. Mithin sind eine Vielzahl an optischen und haptischen Effekten auf überraschend einfache Weise zugänglich. Die jeweils gewünschte Kombination an optischen und/ oder haptischen Eigenschaften des erfindungsgemäßen Nagellacks kann durch geeignete Wahl wenigstens eines Farbmittels und wenigstens eines mikronisierten Wachses eingestellt werden.

Die individuelle Beurteilung des beschriebenen haptischen samtartigen Effekts wird durch taktile Wahrnehmung bestimmt. Der samtartige Effekt kann auch als "soft touch", "soft feel", "non-shiny" oder "satin finish" Effekt bezeichnet werden.

Grundsätzlich kann durch eine Erhöhung des Gewichtsanteils an mikronisiertem Wachs dem erfindungsgemäßen Nagellack nach der Applikation und Trocknung ein stärker ausgeprägter samtartiger Effekt verliehen werden. Die Obergrenze der Zugabe an mikronisiertem Wachs kann durch die Eigenschaften der jeweiligen Nagellackbasis mitbestimmt und kann durch entsprechende Konzentrationsreihen experimentell ermittelt werden. Bei Zugabe einer zu großen Menge an mikronisiertem Wachs kann die Viskosität stark erhöht und damit die Applizierbarkeit auf dem Nagel verschlechtert werden. Erfindungsgemäß enthält der Nagellack mikronisiertes Wachs im Bereich von 0,1 bis 25 Gew.-%, vorzugsweise im Bereich von 1,0 bis 20 Gew.-%, jeweils bezogen auf das Gesamtgewicht des erfindungsgemäßen Nagellackes. Bei einer Kombination mit Effektpigmenten liegt die Menge des wenigstens einen mikronisierten Wachses bevorzugt in einem Bereich von 1 bis 15 Gew.-%, besonders bevorzugt in einem Bereich von 3 bis 10 Gew.-%, jeweils bezogen auf das Gesamtgewicht des erfindungsgemäßen Nagellackes.

Organische und anorganische Pigmente können dem erfindungsgemäßen Nagellack in einem Bereich von 0,05 bis 2,5 Gew.%, bevorzugt in einem Bereich von 0,06 bis 1,0 Gew.-% und besonders bevorzugt in einem Bereich von 0,07 bis 0,5 Gew.-%, jeweils bezogen auf das Gesamtgewicht des erfindungsgemäßen Nagellacks, zugefügt werden.

Organische und anorganische Pigmente werden dem erfindungsgemäßen Nagellack bevorzugt als Dispersionen zugegeben.

Effektpigmente werden im erfindungsgemäßen Nagellack bevorzugt in einem Bereich von 0,05 bis 6,0 Gew.-%, besonders bevorzugt in einem Bereich von 0,1 bis 5,0 Gew.-%, weiter bevorzugt in einem Bereich von 0,15 bis 4 Gew.-% und ganz besonders bevorzugt in einem Bereich von 0,2 bis 3,5 Gew.-%, jeweils bezogen auf das Gesamtgewicht des erfindungsgemäßen Nagellackes, eingesetzt.

Im Unterschied zu Nagellacken ohne mikronisierte(s) Wachs(e) zeichnen sich Nagellacke, welche mikronisierte(s) Wachs(e) enthalten, durch eine optische Tiefenwirkung des applizierten und getrockneten Nagellackfilmes aus. Ein Nagellack, welcher beispielsweise ausschließlich ein Metalleffektpigment und kein mikronisiertes Wachs enthält, zeigt, wie bereits beschrieben, nach der Applikation und Trocknung einen im Wesentlichen winkelabhängigen Glanzeffekt bzw. einen sogenannten Hell-/ Dunkelflop. Diese Helligkeitsänderung wird durch den Flopindex beschrieben. Der Flopindex wird nach Alman folgendermaßen definiert (S. Schellenberger, M. Entenmann, A. Hennemann, P. Thometzek, Farbe und Lack, 04/2007, S. 130):

$$\text{Flopindex} = 2{,}69 \cdot (L_{E1} - L_{E3})^{1{,}11} / L_{E2}^{0{,}86}$$

mit $L_{E1}$ Helligkeit des glanznahen Messwinkels (E1 = 15° zum Glanzwinkel), $L_{E2}$ Helligkeit des Messwinkels zwischen glanznahem und glanzfernen Winkel (E2 = 45° zum Glanzwinkel) und $L_{E3}$ Helligkeit des glanzfernen Messwinkels (E3 = 110° zum Glanzwinkel). Je größer der Zahlenwert des Flopindexes ist, desto stärker kommt der Hell-/ Dunkelflop zum Ausdruck.

Für einen Betrachter ist der optische Eindruck der Nagellackapplikation dabei abhängig von der Teilchengröße sowie von der Art des Metalleffektpigments. Beispielsweise lassen dünne feine Metalleffektpigmente mit einer mittleren Teilchengröße $D_{50}$ aus einem Bereich von 5 bis 27 $\mu$m und einem Aspektverhältnis, d.h. einem Verhältnis von Pigmentdurchmesser zu Pigmentdicke, im Bereich von 50 bis 200 einen lackierten Fingernagel im Glanzwinkel wie flüssiges Metall glänzen. Dieser Effekt kommt bei sehr dünnen, nach PVD-Verfahren hergestellten Metalleffektpigmenten, noch deutlicher zum Ausdruck. Außerhalb des Glanzwinkels erscheint diese Nagellackapplikation einem Betrachter allerdings eher dunkel und glanzlos. Wird einem mit dem PVD-Metalleffektpigment versehenen Nagellack wenigstens ein mikronisiertes Wachs zugesetzt, so nimmt ein Betrachter jetzt nach Applikation und Trocknung überraschenderweise über den gesamten Betrachtungswinkel einen gewissen Glitzereffekt wahr. Im Glanzwinkel kann dann nicht oder nur in deutlich abgeschwächter Form der stärkere metallische Glanz und der markante Hell-/Dunkelflop einer Nagellackapplikation ohne mikronisiertes Wachs wahrgenommen werden. Durch den Zusatz von mikronisiertem Wachs wirkt die erfindungsgemäße Nagellackapplikation insgesamt lebendiger.

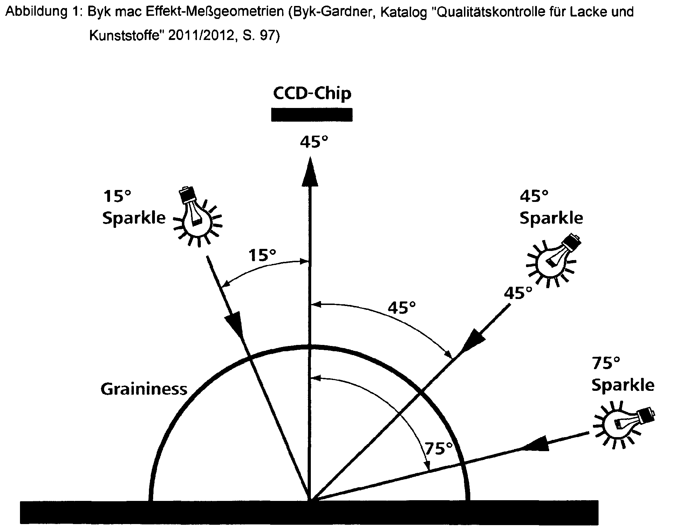

Um den optischen Einfluss des wenigstens einen mikronisierten Wachses in Gegenwart wenigstens eines Effektpigments objektiv beschreiben zu können, wurden Mehrwinkelfarb- und Effektmessungen mit einem BYK-mac (Fa. Byk-Gardner) anhand von Nagellackapplikationen auf Schwarz-Weiß-Deckungskarten (Chart PA-2812, Fa. Byk-Gardner) durchgeführt. Der BYK-mac misst den Gesamtfarbeindruck unter verschiedenen Beobachtungswinkeln und Lichtverhältnissen. Die Mehrwinkelfarbmessung dient hierbei zur Erfassung und Beschreibung des Hell-/ Dunkelflops und/ oder Farbflops von mit Effektpigmenten versehenen Nagellacken. Relativ zum Ausfallwinkel des bei 45° eingestrahlten Lichts werden die Messgeometrien (-15 °), + 15°, 25°, 45°, 75°, 110° gemessen. Zur Simulation von Effektänderungen bei direkter und diffuser Beleuchtung werden gleichzeitig Glitzereffekt und Körnigkeit mit Hilfe einer hochauflösenden CCD-Kamera kontrolliert. Der Glitzereffekt, verursacht durch das Reflexionsvermögen der einzelnen Effektpigmente, wird nur bei direkter Sonneneinstrahlung wahrgenommen und verändert sich in Abhängigkeit des Beleuchtungswinkels. Aus diesem Grund beleuchtet der Byk-mac die Probe mit sehr hellen LEDs unter drei verschiedenen Winkeln (15°/ 45°/ 75°, Abbildung 1). Die CCD-Kamera nimmt dabei senkrecht zur Oberfläche jeweils ein Bild auf. Die Bilder werden mit Hilfe von Bildverarbeitungsalgorithmen analysiert, wobei das Histogramm der Helligkeitsstufen als Basis für die Berechnung der Glitzerparameter verwendet wird. Um eine bessere Differenzierung zu gewährleisten, kann der Glitzereffekt durch ein zweidimensionales System beschrieben werden, der Glitzerfläche S_a und der Glitzerintensität S_i, welche auch in einem eindimensionalen Wert, dem Glitzergrad S_G zusammengefasst werden können (Byk-Gardner, Katalog "Qualitätskontrolle für Lacke und Kunststoffe" 2011/2012, S. 97/ 98).

Die gemessene Glitzerfläche und Glitzerintensität wird durch die Orientierung der Effektpigmente beeinflusst. Ein gut, d.h. weitgehend planparallel zum Untergrund ausgerichtetes Effektpigment weist bei einem Vergleich der in den Beleuchtungsgeometrien 15°, 45° und 75° erhaltenen Glitzermesswerte S_a, S_i, und S_G die höchsten Messwerte bei einer Beleuchtungsgeometrie von 15° auf, da ein großer Teil der Effektpigmente das eingestrahlte Licht direkt reflektiert. Bei einer Beleuchtungsgeometrie von 45° wird das eingestrahlte Licht weitgehend direkt reflektiert und damit bei Beobachtung senkrecht zur Applikation als schwächerer Glitzereffekt wahrgenommen. Der bei dieser Beleuchtungsgeometrie beobachtete Glitzereffekt geht teilweise auf fehl-, d.h. nicht planparallel, orientierte Effektpigmente zurück, die das bei 45° eingestrahlte Licht in Richtung des Detektors ablenken können. Bei einer Beleuchtungsgeometrie von 75° wird senkrecht zur Applikation kein bzw. nur ein schwacher Glitzereffekt wahrgenommen. Dieser Effekt wird wiederum durch fehlgeordnete Effektpigmente bedingt.

Somit weist ein gut orientiertes Effektpigment den größten Glitzereffekt bei 15° auf, der minimale Glitzereffekt relativ zu der 15° Messung wird bei 75° beobachtet. Im Falle eines schlecht orientierten Effektpigments werden die Differenzen der bei 15°, 45° und 75° Beleuchtungsgeometrie beobachteten Messwerte kleiner, da durch die Fehlorientierung stets Licht in Richtung des Detektors reflektiert wird.

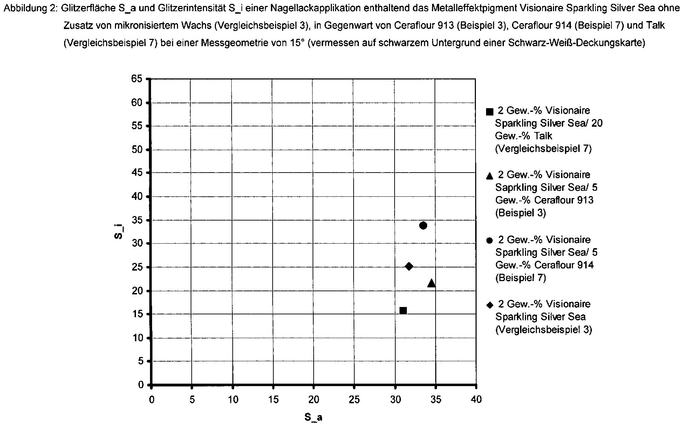

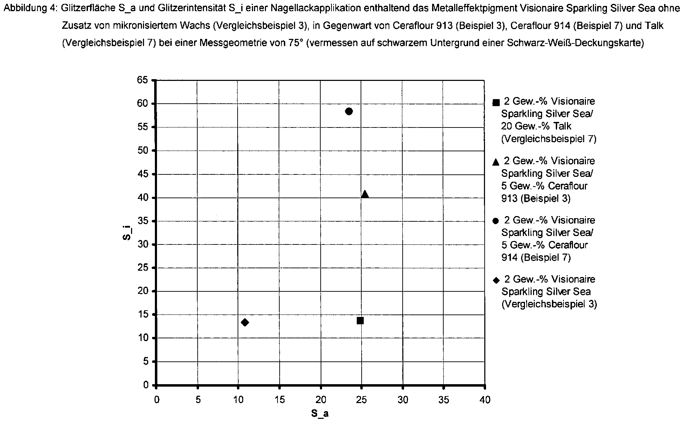

Im Folgenden werden beispielhaft die mit dem BYK-mac gemessenen Glitzerwerte am Beispiel eines groben Metalleffektpigments mit einer mittleren Teilchengröße von 45 bis 71 $\mu$m (Visionaire Sparkling Silver Sea, Fa. Eckart) erläutert (Abbildungen 2 bis 4). Wird die Applikation eines Nagellacks enthaltend ein grobes Metalleffektpigment ohne Zusatz wenigstens eines mikronisierten Wachses auf einer Schwarz-Weiß-Deckungskarte (Chart PA-2812, Fa. Byk-Gardner) vermessen, so wird ausgehend von der 15° Beleuchtungsgeometrie eine zunehmende Abnahme des Glitzergrades S_G beobachtet. Wird die Effektpigmentorientierung jedoch durch Zugabe mindestens eines mikronisierten Wachses gestört, so nähern sich die Messwerte des bei 15°, 45° und 75° Beleuchtungsgeometrie beobachteten Glitzergrades S_G an einander an. Es wird vermutet, dass durch die Zugabe mindestens eines mikronisierten Wachses eine Fehlorientierung des groben Metalleffektpigments induziert wird, so dass eine deutlich wahrnehmbare Zahl an Metalleffektpigmenten das eingestrahlte Licht auf den Detektor reflektiert.

Würde anstelle des wenigstens einen mikronisierten Wachses zur Erzielung eines matt wirkenden Nagellacks ein handelsübliches Mattierungsmittel wie beispielsweise Talk eingesetzt, so müsste zur Erzielung eines vergleichbaren optischen Effekts eine deutlich größere Menge an Mattierungsmittel zugefügt werden. Im Fall von Talk müssten wenigstens 15 Gew.-% eingesetzt werden. Bedingt durch den hohen Anteil an opaken Talkpartikeln geht dann jedoch die Glitzerintensität S_i, verloren.

Maßgeblich für den optischen Eindruck ist der eindimensionale Glitzergrad S_G. Je höher der Zahlenwert von S_G, desto höher ist der auch vom Auge wahrnehmbare Glitzereffekt. In einer zweidimensionalen Darstellung kann der Glitzergrad S_G in die Komponenten Glitzerintensität S_i, i und Glitzerfläche S_a aufgeteilt werden. Da beide Komponenten einen maßgeblichen Einfluss auf den Glitzergrad S G haben, kann es vorkommen, dass ein Effektpigment in den Messgeometrien 15°, 45° und 75° nahezu den gleichen Glitzergrad S_G aufweist, obwohl sich die Zahlenwerte von S_a und S_G in den betrachteten Winkeln deutlich erhöhen bzw. erniedrigen.

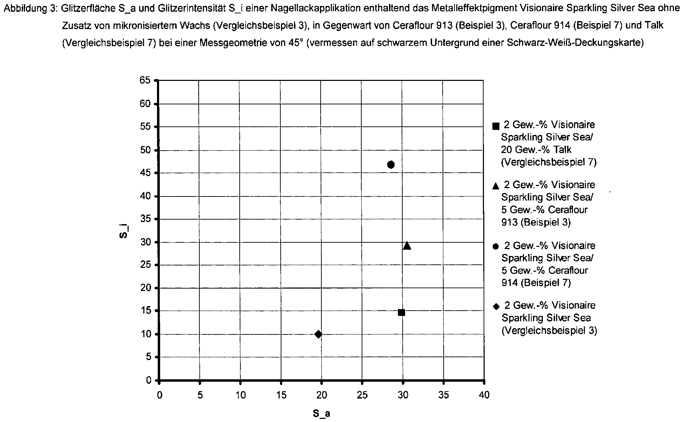

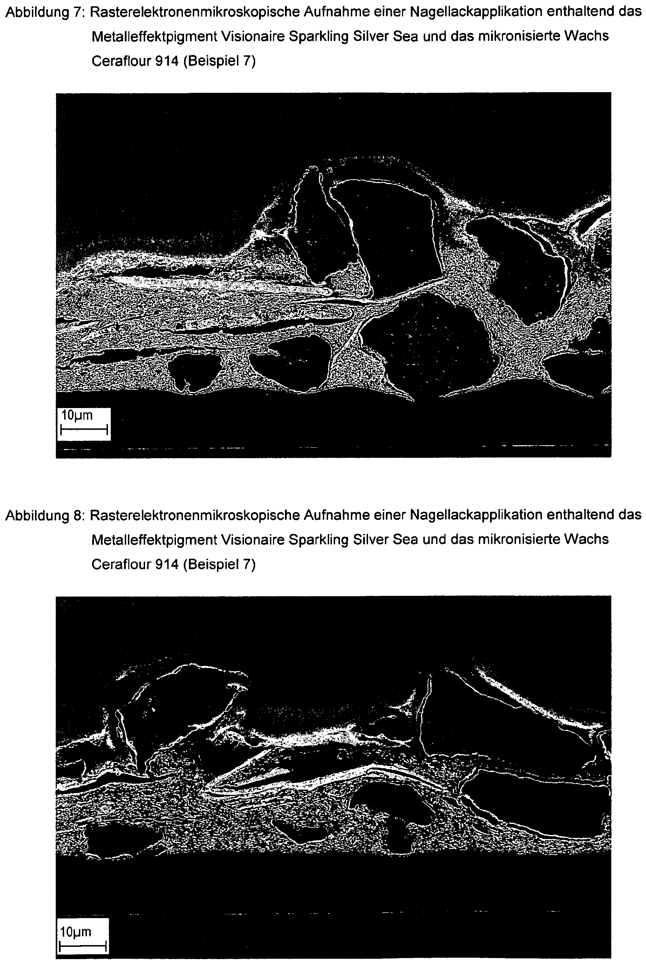

Nagellacke ohne Zusatz von mikronisiertem Wachs erlauben eine weitgehend planparallele Anordnung von plättchenförmigen Effektpigmenten zum jeweiligen Untergrund. Abbildung 2 und Abbildung 3 zeigt eine Rasterelektronenmikroskopaufnahme eines Nagellacks enthaltend ein Metalleffektpigment. Hier ist sehr deutlich die optimale planparallele Orientierung des Metalleffektpigments zu erkennen. In Gegenwart wenigstens eines mikronisierten Wachses ist eine planparallele Anordnung eines plättchenförmigen Effektpigments zum jeweiligen Untergrund nicht immer möglich, so dass die plättchenförmigen Effektpigmente in Abhängigkeit von ihrer eigenen Teilchengröße und der Teilchengröße des wenigstens einen mikronisierten Wachses zu einer mehr oder weniger stark ausgeprägten Fehlanordnung, also einer nicht mehr zum Untergrund parallelen Anordnung, gezwungen werden. In den Abbildungen 7 bis 9 ist eine derartige Fehlstellung eines Metalleffektpigments, verursacht durch die Anwesenheit eines mikronisierten Wachses, anhand einer Rasterelektronenmikroskopaufnahme gezeigt. Durch diese nicht optimale Ausrichtung der plättchenförmigen Effektpigmente geht im Glanzwinkel ein beträchtliches Maß an Glanz verloren. Dafür sind durch diese Fehlanordnung der plättchenförmigen Effektpigmente einem Betrachter aus unterschiedlichen Blickwinkeln immer wieder plättchenförmige Effektpigmente im Glanzwinkel zugewandt, die sich durch ihren Glanz und/ oder Glitzereffekt bemerkbar machen. Ein Betrachter nimmt also durch die Anwesenheit von mikronisiertem Wachs einen verminderten Effekt im Glanzwinkel, dafür einen merklichen Glitzereffekt über einen größeren Winkelbereich wahr.

Darüber hinaus bedingt der Zusatz von wenigstens einem mikronisierten Wachs schon bei einer vergleichsweise geringen Einsatzmenge, beispielsweise < 10 Gew.-%, bezogen auf das Gesamtgewicht des erfindungsgemäßen Nagellacks, den gewünschten samtartigen Effekt, der auch als Mattierungseffekt bezeichnet werden kann. Bei einer Einsatzmenge < 10 Gew.-% an Talk wird zwar eine Abschwächung des metallischen Glanzes erzielt, der applizierte Nagellack weist aber nach wie vor einen winkelabhängigen metallischen Glanzeffekt auf.

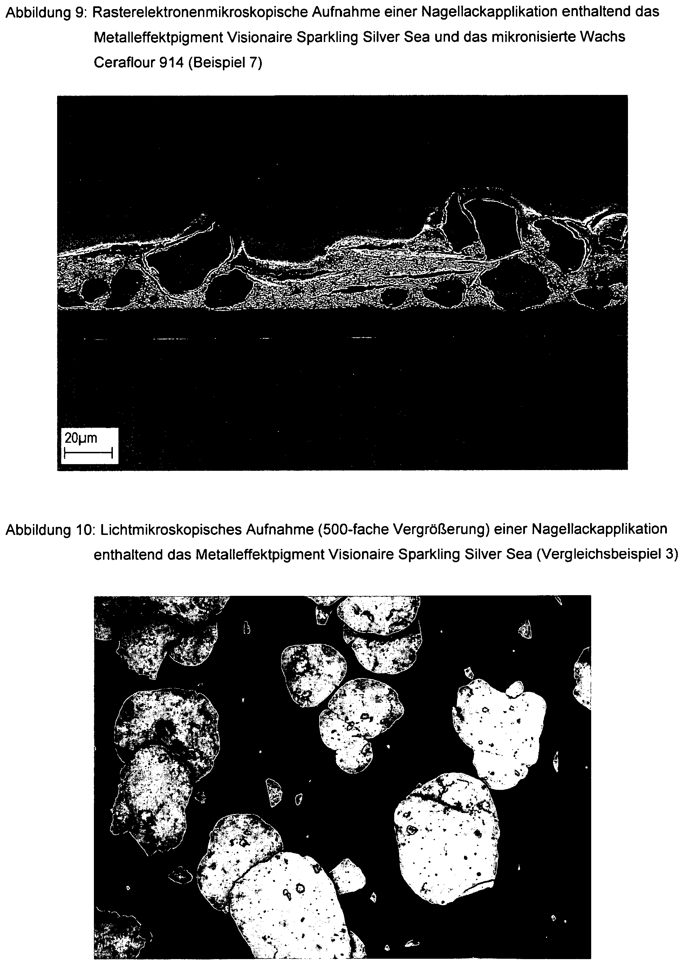

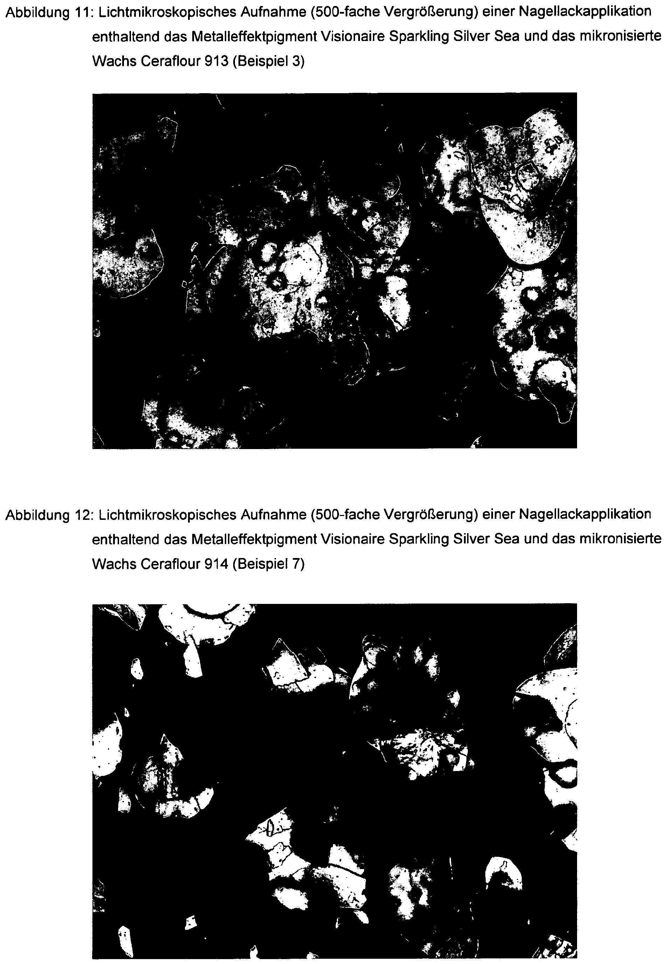

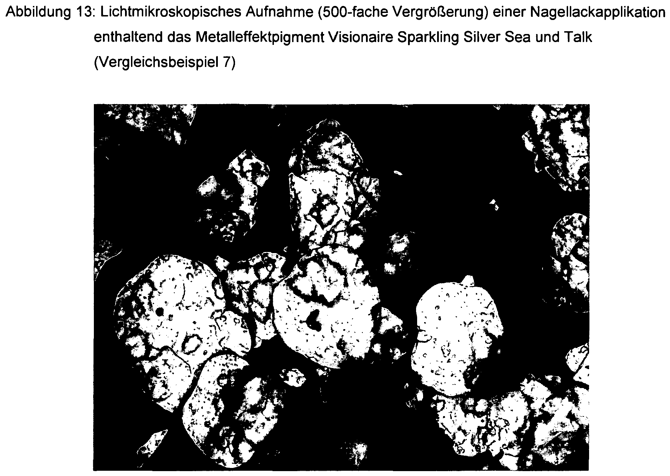

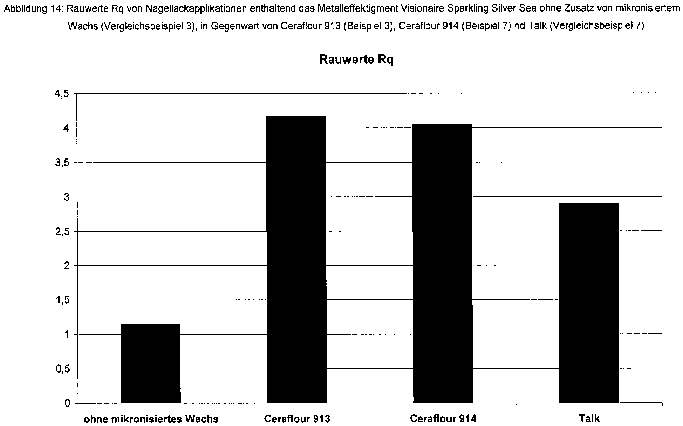

Der haptische Effekt kann auch mit Hilfe lichtmikroskopischer Tiefenprofile der erfindungsgemäßen Nagellacke erfasst werden. Die Nagellackapplikationen wurden hierzu auf Schwarz-Weiß-Deckungskarten (Chart PA-2812, Fa. Byk-Gardner) mit dem Mikroskop Axio Imager.Z1m (Fa. Zeiss) vermessen. Über einen Höhenbereich von ca. 30 $\mu$m senkrecht zur Schwarz-Weiß-Deckungskarte wurden jeweils zwei Mikroskopbilder pro Mikrometer aufgenommen, welche anschließend mit Hilfe der Bildverarbeitungssoftware AxioVision 4.6.3. zu einem einzigen Mikroskopbild (Abbildungen 10 bis 13) übereinandergelegt und gleichzeitig zu einem Topographiebild verrechnet wurden. Aus diesem Topographiebild wurde nach DIN EN ISO 4287:2010-07 die Oberflächenkenngröße "Rauwert" Rq berechnet (Abbildung 14). Als Vergleich diente ein identisch pigmentierter Nagellack ohne Zusatz von mikronisiertem Wachs. Effektpigmente orientieren sich in einem Nagellack in Abwesenheit von mikronisiertem Wachs weitgehend parallel zum Untergrund der Schwarz-Weiß-Deckungskarte, was sich, wie bereits beschrieben, optisch in den hohen Glanz-, Glitzer- und/ oder Helligkeitswerten und haptisch in einer sehr glatten Oberfläche mit entsprechend niedrigen Rauwerten widerspiegelt. Hierbei ist zu beachten, dass die Oberflächengüte zusätzlich durch die Morphologie der über den Effektpigmenten angeordneten Nagellackschicht beeinflusst wird, d.h. die Beurteilung der Haptik muss grundsätzlich in Relation zum jeweils eingesetzten Nagellacksystem erfolgen. Eine unpigmentierte und nicht mit mikronisiertem Wachs versehene Nagellackbasis, die in der Applikation eine eher stumpfe, evtl. sogar sandpapierartige Haptik besitzt, kann durch Zusatz von wenigstens einem mikronisierten Wachs gezielt verändert werden, so dass eine fühlbar geänderte Haptik resultiert. In gleicher Weise wird eine in der Applikation glatte Nagellackbasis durch Zusatz wenigstens eines mikronisierten Wachses in der Haptik modifiziert. Daher muss der Nagellack innerhalb eines solchen Vergleichs selbstverständlich identisch sein. Werden nun mikronisierte Wachse einem mit Effektpigmenten versehenen Nagellack zugegeben, werden die Effektpigmente in ihrer optimalen Orientierung gestört. Die Anwesenheit des mikronisierten Wachses führt dann sowohl im Hinblick auf die Optik als auch die Haptik zu einer Änderung. Im Vergleich zu einem Nagellack ohne mikronisiertes Wachs ist ein deutlicher Anstieg des Rauwerts zu beobachten. Würde anstelle des wenigstens einen mikronisierten Wachses nun Talk eingesetzt werden, so müsste dem Nagellack zur Erzielung eines vergleichbaren Rauwerts eine deutlich höhere Menge an Talk zugesetzt werden. So wird beispielsweise auch durch Zusatz von 20 Gew.-% Talk nach Applikation und Trocknung des Nagellacks der Rauwert eines entsprechenden, aber mit 5 Gew.-% mikronisiertem Wachs versehenen erfindungsgemäßen Nagellacks, nicht erreicht (Abbildung 14).

Die nachfolgenden Beispiele dienen der näheren Beschreibung der Erfindung und sollen in keinerlei Hinsicht einschränkend sein. Die nachstehenden Kombinationsmöglichkeiten von wenigstens einem Farbmittel und wenigstens einem mikronisierten Wachs stellen nur eine beispielhafte Auswahl von möglichen Nagellackzusammensetzungen dar.

**I Herstellung der erfindungsgemäßen Nagellacke**

Erfindungsgemäße Beispiele 1 bis 68
Phase A

| Beispiel | INCI Name | Produktname | Gew-% |
|---|---|---|---|
| 1 | CI 77 000 (Aluminum Powder), Silica | Visionaire Bright Silver Sea[1] | 2,00 |
| | | Ceraflour 913[2] | 5,00 |
| 2 | CI 77 000 (Aluminum Powder), Silica | Visionaire Bright Silver Sea | 2,00 |
| | | Ceraflour 914[2] | 5,00 |
| 3 | CI 77 000 (Aluminum Powder), Silica | Visionaire Sparkling Silver Sea[1] | 2,00 |
| | | Ceraflour 913 | 5,00 |
| 4 | CI 77 000 (Aluminum Powder), Silica | Visionaire Sparkling Silver Sea | 2,00 |
| | | Ceraflour 913 | 10,00 |
| 5 | CI 77 000 (Aluminum Powder), Silica | Visionaire Sparkling Silver Sea | 2,00 |
| | | Ceraflour 913 | 15,00 |
| 6 | CI 77 000 (Aluminum Powder), Silica | Visionaire Sparkling Silver Sea | 2,00 |
| | | Ceraflour 913 | 20,00 |
| 7 | CI 77 000 (Aluminum Powder), Silica | Visionaire Sparkling Silver Sea | 2,00 |
| | | Ceraflour 914 | 5,00 |
| 8 | CI 77 000 (Aluminum Powder), Silica | Visionaire Sparkling Silver Sea | 2,00 |
| | | Ceraflour 914 | 10,00 |

(fortgesetzt)

| Beispiel | INCI Name | Produktname | Gew-% |
|---|---|---|---|
| 9 | CI 77 000 (Aluminum Powder), Silica | Visionaire Sparkling Silver Sea | 2,00 |
| | | Ceraflour 914 | 15,00 |
| 10 | CI 77 000 (Aluminum Powder), Silica | Visionaire Sparkling Silver Sea | 2,00 |
| | | Ceraflour 914 | 20,00 |
| 11 | CI 77 000 (Aluminum Powder), Silica | Visionaire Sparkling Silver Sea | 0,50 |
| | | Ceraflour 915[2] | 5,00 |
| 12 | CI 77 000 (Aluminum Powder), Silica | Visionaire Sparkling Silver | 2,00 |
| | | Sea Ceraflour 915 | 5,00 |
| 13 | CI 77 000 (Aluminum Powder), Silica | Visionaire Sparkling Silver Sea | 2,00 |
| | | Ceraflour 916[2] | 5,00 |
| 14 | CI 77 000 (Aluminum Powder), Silica | Visionaire Sparkling Silver Sea | 2,00 |
| | | Ceraflour 990[2] | 5,00 |
| 15 | CI 77 000 (Aluminum Powder), Silica | Visionaire Sparkling Silver Sea | 2,00 |
| | | Ceraflour 991[2] | 5,00 |
| 16 | CI 77 000 (Aluminum Powder), Silica | Visionaire Sparkling Silver Sea | 1,00 |
| | Calcium Sodium Borosilicate, CI 77 891 (Titanium Dioxide), Tin Oxide | Mirage Glamour Red[1] | 1,00 |
| | | Ceraflour 913 | 5,00 |
| 17 | CI 77 000 (Aluminum Powder), Silica | Visionaire Sparkling Silver Sea | 1,00 |
| | Calcium Sodium Borosilicate, CI 77 891 (Titanium Dioxide), Tin Oxide | Mirage Glamour Red | 1,00 |
| | | Ceraflour 914 | 5,00 |
| 18 | CI 77 000 (Aluminium Powder), Isopropyl Alcohol | Silverdream Moonlight 50IL[1] | 2,00 |
| | | Ceraflour 913 | 5,00 |
| 19 | CI 77 000 (Aluminium Powder), Isopropyl Alcohol | Silverdream Moonlight 50IL | 2,00 |
| | | Ceraflour 914 | 5,00 |
| 20 | Iron Powder, Paraffinum Liquidum (Mineral Oil) | Stapa WM Iron VP 401040[1] | 2,00 |
| | | Ceraflour 913 | 5,00 |
| 21 | Iron Powder, Paraffinum Liquidum (Mineral Oil) | Stapa WM Iron VP 401040 | 2,00 |
| | | Ceraflour 913 | 10,00 |
| 22 | Iron Powder, Paraffinum Liquidum (Mineral Oil) | Stapa WM Iron VP 01040 | 2,00 |
| | | Ceraflour 913 | 15,00 |

(fortgesetzt)

| Beispiel | INCI Name | Produktname | Gew-% |
|---|---|---|---|
| 23 | Iron Powder, Paraffinum Liquidum (Mineral Oil) | Stapa WM Iron VP 401040 | 2,00 |
| | | Ceraflour 913 | 20,00 |
| 24 | Iron Powder, Paraffinum Liquidum (Mineral Oil) | Stapa WM Iron VP 401040 | 2,00 |
| | | Ceraflour 914 | 5,00 |
| 25 | Iron Powder, Paraffinum Liquidum (Mineral Oil) | Stapa WM Iron VP 401040 | 2,00 |
| | | Ceraflour 914 | 10,00 |
| 26 | Iron Powder, Paraffinum Liquidum (Mineral Oil) | Stapa WM Iron VP 401040 | 2,00 |
| | | Ceraflour 914 | 15,00 |
| 27 | Iron Powder, Paraffinum Liquidum (Mineral Oil) | Stapa WM Iron VP 401040 | 2,00 |
| | | Ceraflour 914 | 20,00 |
| 28 | Mica, CI 77 891 (Titanium Dioxide) | Prestige Super Soft Silver | 2,00 |
| | | Ceraflour 913 | 5,00 |
| 29 | Mica, CI 77 891 (Titanium Dioxide) | Prestige Super Soft Silver | 2,00 |
| | | Ceraflour 914 | 5,00 |
| 30 | Mica, CI 77 891 (Titanium Dioxide) | Prestige Super Soft Silver | 2,00 |
| | Polyethylene | Micropoly 250S[3] | 5,00 |
| 31 | Mica, CI 77 891 (Titanium Dioxide) | Prestige Super Soft Silver | 2,00 |
| | Polethylene/ PTFE | Microsilk 418[3] | 5,00 |
| 32 | Mica, CI 77 891 (Titanium Dioxide) | Dioxide) Prestige Super Soft Silver 2,00 | 2,00 |
| | Polypropyiene/ PTFE | Microsilk 920[3] | 5,00 |
| 33 | Mica, CI 77 891 (Titanium Dioxide) | Prestige Super Soft Silver | 2,00 |
| | PTFE | Microslip 519[3] | 5,00 |
| 34 | Mica, CI 77 891 (Titanium Dioxide), Tin Oxide | Prestige Silver Star[1] | 2,00 |
| | | Ceraflour 913 | 5,00 |
| 35 | Mica, CI 77 891 (Titanium Dioxide), Tin Oxide | Prestige Silver Star | 2,00 |
| | | Ceraflour 914 | 5,00 |
| 36 | Mica, CI 77 891 (Titanium Dioxide), Tin Oxide | Prestige Sparkling Silver Star[1] | 2,00 |
| | | Ceraflour 913 | 5,00 |
| 37 | Mica, CI 77 891 (Titanium Dioxide), Tin Oxide | Prestige Sparkling Silver Star | 2,00 |
| | | Ceraflour 914 | 5,00 |
| 38 | Synthetic Fluorophlogopite, CI 77 891 (Titanium Dioxide), Tin Oxide | SynCrystal Silver[1] | 2,00 |
| | | Ceraflour 913 | 5,00 |
| 39 | Synthetic Fluorophlogopite, CI 77 891 (Titanium Dioxide), 39 Tin Oxide | SynCrystal Silver | 2,00 |
| | | Ceraflour 914 | 5,00 |

(fortgesetzt)

| Beispiel | INCI Name | Produktname | Gew-% |
|---|---|---|---|
| 40 | Synthetic Fluorophlogopite, CI 77 891 (Titanium Dioxide), Tin Oxide | SynCrystal Sparkling Silver[1] | 2,00 |
| | | Ceraflour 913 | 5,00 |
| 41 | Synthetic Fluorophlogopite, CI 77 891 (Titanium Dioxide), Tin Oxide | SynCrystal Sparkling Silver | 2,00 |
| | | Ceraflour 914 | 5,00 |
| 42 | Calcium Sodium Borosilicate, CI 77 891 (Titanium Dioxide), Tin Oxide | Mirage Bright Silver[1] | 2,00 |
| | | Ceraflour 913 | 5,00 |
| 43 | Calcium Sodium Borosilicate, CI 77 891 (Titanium Dioxide), Tin Oxide | Mirage Bright Silver | 2,00 |
| | | Ceraflour 914 | 5,00 |
| 44 | Calcium Sodium Borosilicate, CI 77 891 (Titanium Dioxide), Tin Oxide | Mirage Bright Silver | 2,00 |
| | | Ceraflour 915 | 1,00 |
| 45 | Calcium Sodium Borosilicate, CI 77 891 (Titanium Dioxide), Tin Oxide | Mirage Bright Silver | 2,00 |
| | | Ceraflour 915 | 2,00 |
| 46 | Calcium Sodium Borosilicate, CI 77 891 (Titanium Dioxide), Tin Oxide | Mirage Bright Silver | 2,00 |
| | | Ceraflour 915 | 3,00 |
| 47 | Calcium Sodium Borosilicate, CI 77 891 (Titanium Dioxide), Tin Oxide | Mirage Bright Silver | 2,00 |
| | | Ceraflour 915 | 4,00 |
| 48 | Calcium Sodium Borosilicate, CI 77 891 (Titanium Dioxide), Tin Oxide | Mirage Bright Silver | 2,00 |
| | | Ceraflour 915 | 5,00 |
| 49 | Calcium Sodium Borosilicate, CI 77 891 (Titanium Dioxide), Tin Oxide | Mirage Bright Silver | 2,00 |
| | Polyethylene | Micropoly 250S | 5,00 |
| 50 | Calcium Sodium Borosilicate, CI 77 891 (Titanium Dioxide), Tin 50 Oxide | Mirage Bright Silver | 2,00 |
| | Polethylene/ PTFE Microsilk | Microsilk 418 | 5,00 |
| 51 | Calcium Sodium Borosilicate, CI 77 891 (Titanium Dioxide), Tin Oxide | Mirage Bright Silver | 2,00 |
| | Polypropylene/ PTFE | Microsilk 920 | 5,00 |
| 52 | Calcium Sodium Borosilicate, CI 77 891 (Titanium Dioxide), Tin Oxide | Mirage Bright Silver | 2,00 |
| | PTFE | Microslip 519 | 5,00 |
| 53 | Calcium Sodium Borosilicate, CI 77 891 (Titanium Dioxide), Tin Oxide | Mirage Bright Blue[1] | 2,00 |
| | | Ceraflour 913 | 5,00 |

(fortgesetzt)

| Beispiel | INCI Name | Produktname | Gew-% |
|---|---|---|---|
| 54 | Calcium Sodium Borosilicate, CI 77 891 (Titanium Dioxide), Tin Oxide | Mirage Bright Blue | 2,00 |
| | | Ceraflour 914 | 5,00 |
| 55 | Calcium Sodium Borosilicate, CI 77 891 (Titanium Dioxide), Tin Oxide | Mirage Sparkling Silver[1] | 2,00 |
| | | Ceraflour 913 | 5,00 |
| 56 | Calcium Sodium Borosilicate, CI 77 891 (Titanium Dioxide), Tin Oxide | Mirage Sparkling Silver | 2,00 |
| | | Ceraflour 913 | 7,00 |
| 57 | Calcium Sodium Borosilicate, CI 77 891 (Titanium Dioxide), Tin Oxide Silver | Mirage Sparkling Silver | 2,00 |
| | | Ceraflour 913 | 10,00 |
| 58 | Calcium Sodium Borosilicate, CI 77 891 (Titanium Dioxide), Tin Oxide | Mirage Sparkling Silver | 2,00 |
| | | Ceraflour 913 | 15,00 |
| 59 | Calcium Sodium Borosilicate, CI 77 891 (Titanium Dioxide), Tin Oxide | Mirage Sparkling Silver | 2,00 |
| | | Ceraflour 914 | 5,00 |
| 60 | Calcium Sodium Borosilicate, CI 77 891 (Titanium Dioxide), Tin Oxide | Mirage Sparkling Silver | 2,00 |
| | | Ceraflour 914 | 7,00 |
| 61 | Calcium Sodium Borosilicate, CI 77 891 (Titanium Dioxide), Tin Oxide | Mirage Sparkling Silver | 2,00 |
| | | Ceraflour 914 | 10,00 |
| 62 | Calcium Sodium Borosilicate, CI 77 891 (Titanium Dioxide), Tin Oxide | Mirage Sparkling Silver | 2,00 |
| | | Ceraflour 914 | 15,00 |
| 63 | Calcium Sodium Borosilicate, CI 77 891 (Titanium Dioxide), CI 77 492 (Iron Oxides), Silica, Tin Oxide | Mirage Sparkling Luxury Gold[1] | 2,00 |
| | | Ceraflour 913 | 5,00 |
| 64 | Calcium Sodium Borosilicate, CI 77 891 (Titanium Dioxide), CI 77492 (Iron Oxides), Silica , Tin Oxide | Mirage Sparkling Luxury Gold | 2,00 |
| | | Ceraflour 914 | 5,00 |
| 65 | Calcium Sodium Borosilicate, CI 77 891 (Titanium Dioxide), Tin Oxide | Mirage Glamour Silver[1] | 2,00 |
| | | Ceraflour 913 | 5,00 |
| 66 | Calcium Sodium Borosilicate, CI 77 891 (Titanium Dioxide), Tin Oxide | Mirage Glamour Silver | 2,00 |
| | | Ceraflour 914 | 5,00 |
| 67 | Calcium Sodium Borosilicate, CI 77 891 (Titanium Dioxide), Tin Oxide | Mirage Twinkling Silver[1] | 2,00 |
| | | Ceraflour 913 | 5,00 |

(fortgesetzt)

| Beispiel | INCI Name | Produktname | Gew-% |
|---|---|---|---|
| 68 | Calcium Sodium Borosilicate, Cl 77 891 (Titanium Dioxide), Tin Oxide | Mirage Twinkling Silver | 2,00 |
| | | Ceraflour 914 | 5,00 |
| [1] Hersteller/ Lieferant: Fa. Eckart<br>[2] Hersteller/ Lieferant: Fa. Byk-Chemie | | | |

| Mikronisiertes Wachs | $D_{50}$ (μm) |
|---|---|
| Ceraflour 913 | 18 |
| Ceraflour 914 | 24 |
| Ceraflour 915 | 34 |
| Ceraflour 916 | 46 |
| Ceraflour 990 | 6 |
| Ceraflour 991 | 5 |
| [3] Hersteller: Fa. Micro Powders | |

| Mikronisiertes Wachs | $D_{50}$ (μm) |
|---|---|
| Micropoly 250S | 2,0 - 4,0 |
| Microsilk 418 | 10,0 - 12,0 |
| Microsilk 920 | 7,0 - 11,0 |
| Microslip 519 | 5,0 - 6,0 |

Phase B

a) Kommerziell erhältliche Nagellackbasis

| INCI Name | Produktname | Gew.% | Hersteller/ Lieferant |
|---|---|---|---|
| Butylacetate, Ethylacetate, Nitrocellulose, Isopropyl Alcohol | International Lacquers Nailpolish & Care Base 359 | Ad 100,00 | Fa. International Lacquers |

b) Alternativ kann die Phase B aus beispielsweise folgenden Bestandteilen hergestellt werden:

| INCI Name | Produktname | Gew.-% | Hersteller/ Lieferant |
|---|---|---|---|
| Phase A' | | | |
| Butyl acetate | n-Butylacetat | 34 | Fa. VWR |
| Ethyl acetate | Ethylacetat | 20 | Fa. VWR |
| Nitrocellulose, Isopropyl alcohol | Walsroder Nitrocellulose E400 Isopropanol 30 % | 23 | Fa. Dow Wolff Cellulosics |
| Phase B' | | | |

(fortgesetzt)

| INCI Name | Produktname | Gew.-% | Hersteller/ Lieferant |
|---|---|---|---|
| Toluenesulfonamide, n-Butyl acetate | Ketjenflex MS80 | 10 | Fa. Axcentive |
| Isopropyl alcohol | 2-Propanol | 5 | Fa. VWR |
| Acetyl tributyl citrate | Tributylacetylcitrat | 7 | Fa. VWR |
| Silica | Aerosil 200 | 1 | Fa. Evonik |

Phase A' und Phase B' werden separat gemischt und anschließend in ein geeignetes Gefäß abgefüllt.

Optional Phase C

Die Einfärbung der Nagellackbasis erfolgte, je nach gewünschtem optischen Effekt, mit wenigstens einem der folgenden Farbstoffe Blanc Covachip W 9705 ET, Jaune Covachip W 1701 ET, Orange Covachip W 2702 ET, Rouge Covachip W 3714 ET, Cerise Covachip W 4702 ET, Rubis Covachip W 4705 ET, Rubis Covachip W 4700 ET, Bleu Covachip W 6709 ET, Brun Covachip W 8700 ET oder Noir Covachip W 9701 ET (Fa. LCW), welche den vereinigten Phasen A und B als 10 Gew.-%ige Dispersion zugefügt wurde. Phase C, die 10 Gew.%ige Dispersion, besteht aus 10 Gew.-% "Covachip", 20 Gew.% Butylacetat, 70 Gew.-% Nagellackbasis (Phase B). Die optionale Phase C kann in dem erfindungsgemäßen Nagellack in einem Bereich von 0,1 bis 10 Gew.-% eingesetzt werden. Entsprechend muss dann die Menge an Nagellackbasis (Phase B) angepasst werden.

Zur Herstellung des erfindungsgemäßen Nagellacks wurden Phase A und Phase B sowie optional Phase C miteinander gemischt und anschließend in ein Nagellackbehältnis abgefüllt. Die optional vorhandene Phase C kann hierbei sowohl zunächst mit Phase B vermischt werden als auch zu den vereinigten Phasen A und B zugegeben werden.

Das mikronisierte Wachs kann in dem erfindungsgemäßen Nagellack in einem Bereich von 0,1 bis 25,0 Gew.-%, vorzugsweise in einem Bereich von 1,0 bis 20 Gew.-%, jeweils bezogen auf das Gesamtgewicht des Nagellacks, eingesetzt werden. Bei Kombination mit Effektpigmenten wird das mikronisierte Wachs bevorzugt in einem Bereich von 1 bis 15 Gew.-%, besonders bevorzugt in einem Bereich von 3 bis 10 Gew.-%, jeweils bezogen auf das Gesamtgewicht des Nagellacks, verwendet. Derartige Variationen können durch eine entsprechende Erhöhung oder Reduzierung der eingesetzten Nagellackbasis (Phase B) ausgeglichen werden.

Organische und anorganische Pigmente können dem erfindungsgemäßen Nagellack in einem Bereich von 0,05 bis 2,5 Gew.%, bevorzugt in einem Bereich von 0,06 bis 1,0 Gew.-% und besonders bevorzugt in einem Bereich von 0,07 bis 0,5 Gew.-%, jeweils bezogen auf das Gesamtgewicht des erfindungsgemäßen Nagellacks, zugefügt werden. Organische und anorganische Pigmente werden dem erfindungsgemäßen Nagellack bevorzugt als Dispersionen zugegeben. Die Zusammensetzung solcher Dispersionen ist beispielhaft bei Phase C beschrieben.

Die Effektpigmente werden im erfindungsgemäßen Nagellack bevorzugt in einem Bereich von 0,05 bis 6,0 Gew.-%, besonders bevorzugt in einem Bereich von 0,1 bis 5,0 Gew.%, weiter bevorzugt in einem Bereich von 0,15 bis 4 Gew.% und ganz besonders bevorzugt in einem Bereich von 0,2 bis 3,5 Gew.-%, jeweils bezogen auf das Gesamtgewicht des erfindungsgemäßen Nagellackes, eingesetzt. Auch diese Variationen können durch eine entsprechende Erhöhung oder Reduzierung der eingesetzten Nagellackbasis ausgeglichen werden.

Vergleichsbeispiele 1 bis 32

Phase A

| Vergleichsbeispiel | INCI Name | Produktname | Gew-% |
|---|---|---|---|
| 1 | CI 77 000 (Aluminum Powder), Silica | Visionaire Bright Silver Sea | 2,00 |
| 2 | CI 77000 (Aluminum Powder), Silica | Visionaire Bright Silver Sea | 2,00 |
| | Talc | Talk[4] | 20,00 |
| 3 | CI 77000 (Aluminum Powder), Silica | Visionaire Sparkling Silver Sea | 2,00 |

(fortgesetzt)

| Vergleichsbeispiel | INCI Name | Produktname | Gew-% |
|---|---|---|---|
| 4 | CI 77 000 (Aluminum Powder), Silica | Visionaire Sparkling Silver Sea | 2,00 |
| | Talc | Talk | 5,00 |
| 5 | CI 77 000 (Aluminum Powder), Silica | Visionaire Sparkling Silver Sea | 2,00 |
| | Talc | Talk | 10,00 |
| 6 | CI 77 000 (Aluminum Powder), Silica | Visionaire Sparkling Silver Sea | 2,00 |
| | Talc | Talk | 15,00 |
| 7 | CI 77 000 (Aluminum Powder), Silica | Visionaire Sparkling Silver Sea | 2,00 |
| | Talc | Talk | 20,00 |
| 8 | CI 77 000 (Aluminum Powder), Silica | Visionaire Sparkling Silver Sea | 1,00 |
| | Calcium Sodium Borosilicate (and) Titanium Dioxide (and) Tin Oxide | Mirage Glamour Red | 1,00 |
| 9 | CI 77 000 (Aluminum Powder), Silica | Visionaire Sparkling Silver Sea | 1,00 |
| | Calcium Sodium Borosilicate (and) Titanium Dioxide (and)Tin Oxide | Mirage Glamour Red | 1,00 |
| | Talc | Talk | 20,00 |
| 10 | CI 77 000 (Aluminium Powder), Isopropyl Alcohol | Silverdream Moonliqht 501L | 2,00 |
| 11 | CI 77 000 (Aluminium Powder), Isopropyl Alcohol | Silverdream Moonlight 501L | 2,00 |
| | Talc | Talk | 20,00 |
| 12 | Iron Powder, Paraffinum Liquidum (Mineral Oil) | Stapa WM Iron VP 401040 | 2,00 |
| 13 | Iron Powder, Paraffinum Liquidum (Mineral Oil) | Stapa WM Iron VP 401040 | 2,00 |
| | Talc | Talk | 5,00 |
| 14 | Iron Powder, Paraffinum Liquidum (Mineral Oil) | Stapa WM Iron VP 401040 | 2,00 |
| | Talc | Talk | 10,00 |
| 15 | Iron Powder, Paraffinum Liquidum (Mineral Oil) | Stapa WM Iron VP 401040 | 2,00 |
| | Talc | Talk | 15,00 |
| 16 | Iron Powder, Paraffinum Liquidum (Mineral Oil) | Stapa WM Iron VP 401040 | 2,00 |
| | Talc | Talk | 20,00 |
| 17 | Mica, CI 77 891 (Titanium Dioxide) | Prestige Super Soft Silver | 2,00 |

(fortgesetzt)

| Vergleichsbeispiel | INCI Name | Produktname | Gew-% |
|---|---|---|---|
| 18 | Mica, CI 77 891 (Titanium Dioxide) | Prestige Super Soft Silver | 2,00 |
| | Polyethylene Microscrub | Microscrub 20PC[3] | 5,00 |
| 19 | Mica, CI 77 891 (Titanium Dioxide), Tin Oxide | Prestige Silver Star | 2,00 |
| 20 | Mica, CI 77 891 (Titanium Dioxide), Tin Oxide | Prestige Sparkling Silver Star | 2,00 |
| 21 | Synthetic Fluorophlogopite, CI 77 891 (Titanium Dioxide), Tin Oxide | SynCrystal Silver | 2,00 |
| 22 | Synthetic Fluorophlogopite, CI 77 891 (Titanium Oxide Silver | SynCrystal Silver | 2,00 |
| | Talc | Talk | 20,00 |
| 23 | Synthetic Fluorophlogopite, CI 77 891 (Titanium Dioxide), Tin Oxide | SynCrystal Sparkling Silver | 2,00 |
| 24 | Synthetic Fluorophlogopite, CI 77 891 (Titanium Dioxide), Tin Oxide | SynCrystal Sparkling Silver | 2,00 |
| | Talc | Talk | 20,00 |
| 25 | Calcium Sodium Borosilicate, CI 77 891 (Titanium Dioxide), Tin Oxide | Mirage Bright Silver | 2,00 |
| 26 | Calcium Sodium Borosilicate, CI 77 891 (Titanium Dioxide) Tin Oxide | Mirage Bright Silver | 2,00 |
| | Polyethylene | Microscrub 20PC | 5,00 |
| 27 | Calcium Sodium Borosilicate, CI 77 891 (Titanium Dioxide), Tin Oxide | Mirage Bright Blue | 2,00 |
| 28 | Calcium Sodium Borosilicate, CI 77 891 (Titanium Dioxide), Tin Oxide | Mirage Bright Blue | 2,00 |
| | Talc | Talk | 20,00 |
| 29 | Calcium Sodium Borosilicate, CI 77 891 (Titanium Dioxide), Tin Oxide | Mirage Sparklinq Silver | 2,00 |
| 30 | Calcium Sodium Borosilicate, CI 77 891 (Titanium Dioxide), CI 77 492 (Iron Oxides), Silica, Tin Oxide | Mirage Sparkling Luxury Gold | 2,00 |
| 31 | Calcium Sodium Borosilicate, CI 77 891 (Titanium Dioxide), Tin Oxide | Mirage Glamour Silver | 2.00 |
| 32 | Calcium Sodium Borosilicate, CI 77 891 (Titanium Dioxide), Tin Oxide | Mirage Twinkling Silver | 2.00 |

[3] Hersteller/ Lieferant: Fa. Micro Powders (Microscrub 20PC mesh size maximum 20, entspricht ca. 841 $\mu$m)
[4] Hersteller/ Lieferant: Fa. BDH Prolabo ($D_{50}$ = 18 $\mu$m)

Phase B

| INCI Name | Produktname | Gew.-% | Hersteller/ Lieferant |
|---|---|---|---|
| Butylacetate, Ethylacetate, Nitrocellulose, Isopropyl Alcohol | International Lacquers Nailpolish & Care Base 359 | Ad 100 | Fa. International Lacquers |

Optional Phase C, analog Phase C der erfindungsgemäßen Beispiele.

Zur Herstellung des erfindungsgemäßen Nagellacks wurden Phase A und Phase B sowie optional Phase C miteinander gemischt und anschließend in ein Nagellackbehältnis abgefüllt.

Mikronisierte Wachse mit einer mittleren Teilchengröße > 200 μm sind für eine Anwendung im erfindungsgemäßen Nagellack weniger geeignet. Das in Vergleichsbeispiel 18 eingesetzte mikronisierte Wachs Microscrub 20PC wurde in der Nagellackapplikation optisch als Fehllackierung wahrgenommen. In der Haptik wurde diese Nagellackapplikation als unangenehm empfunden.

**II Charakterisierung der eingesetzten Pigmente und mikronisierten Wachse**

IIa Teilchengrößenmessung

Die Größenverteilungskurve der Perlglanzpigmente und mikronisierten Wachse wurde mit einem Gerät der Fa. Malvern (Gerät: MALVERN Mastersizer 2000) gemäß Herstellerangaben bestimmt. Hierzu wurden ca. 0,1 g des entsprechenden mikronisierten Wachses oder Perlglanzpigmentes als wässrige Suspension, ohne Zusatz von Dispergierhilfsmitteln, unter ständigem Rühren mittels einer Pasteurpipette in die Probenvorbereitungszelle des Messgerätes gegeben und mehrmals vermessen. Aus den einzelnen Messergebnissen wurden die resultierenden Mittelwerte gebildet. Die Auswertung der Streulichtsignale erfolgte dabei nach der Mie-Theorie, welche auch Brechungs- und Absorptionsverhalten der mikronisierten Wachse bzw. der Pigmente beinhaltet.

Die Größenverteilungskurve der Metalleffektpigmente (in Pulverform) wurde mit einem Gerät der Fa. Sympatec (Gerät: HELOS Partikelgrößenanalyse mit RODOS Trockendispergierung und Dosieraggregat VIBRI) gemäß Herstellerangaben bestimmt. Hierzu wurden je nach Beschaffenheit des zu messenden Metalleffektpigmentes ca. 5 bis 25 cm$^3$ Pulver in das Dosieraggregat gegeben. Nach dem Start des Messvorganges erfolgen dann Dosierung, Trockendispergierung und Messung der Probe durch Laserbeugung automatisch. Die Auswertung der Streulichtsignale erfolgte dabei nach der Fraunhofer-Methode.

Die Größenverteilungskurve der Metalleffektpigmente (in Pastenform), der organischen und anorganischen Pigmente wurde mit einem Gerät der Fa. Quantachrome (Gerät: Cilas 1064) gemäß Herstellerangaben vermessen. Hierzu wurden ca. 50 ml des entsprechenden Pigmentes in Isopropanol suspendiert, 300 Sekunden im Ultraschallbad (Gerät: Sonorex IK 52, Fa. Bandelin) behandelt und anschließend mittels einer Pasteurpipette in die Probenvorbereitungszelle des Messgerätes gegeben und mehrmals vermessen. Aus den einzelnen Messergebnissen wurden die resultierenden Mittelwerte gebildet. Die Auswertung der Streulichtsignale erfolgte dabei nach der Fraunhofer-Methode (Metalleffektpigmente) bzw. der Mie-Theorie (organische und anorganische Pigmente).

Unter der mittleren Größe $D_{50}$ wird im Rahmen dieser Erfindung der $D_{50}$-Wert der Summenhäufigkeitsverteilung der volumengemittelten Größenverteilungsfunktion, wie sie durch Laserbeugungsmethoden erhalten werden, verstanden. Der $D_{50}$-Wert gibt an, dass 50 % der mikronisierten Wachse bzw. Pigmente einen Durchmesser aufweisen, der gleich oder kleiner als der angegebene Wert, beispielsweise 20 μm, ist.

Entsprechend gibt der $D_{90}$-Wert an, dass 90% der mikronisierten Wachse bzw. Pigmente einen Durchmesser aufweisen, der gleich oder kleiner des jeweiligen Wertes ist.

Weiterhin gibt der $D_{10}$-Wert an, dass 10% der mikronisierten Wachse bzw. Pigmente einen Durchmesser aufweisen, der gleich oder kleiner des jeweiligen Wertes ist.

IIb Bestimmung des Schmelzbereichs der mikronisierten Wachse

Der Schmelzbereich wurde mit einem Schmelzpunktmessgerät (Modell 5A 6797, Fa. Gallenkamp) bestimmt. Das Verfahren entsprach der DGF Einheitsmethode C-IV 3a.

| Mikronisiertes Wachs | Schmelzbereich (°C) |
|---|---|
| Ceraflour 913 | 158 - 160 |
| Ceraflour 914 | 156 - 160 |
| Micropoly 250S | 129 - 130 |
| Microsilk 418 | 116 - 118 |

**III Charakterisierung des optischen Effekts der erfindungsgemäßen Nagellacke**

IIIa Bestimmung des Hell-/ Dunkelflops (Flopindex)

Der Hell-/ Dunkelflop (Flopindex) von mit Effektpigmenten versehenen Nagellacken wurde anhand von Nagellackapplikationen auf Schwarz-Weiß-Deckungskarten (Chart PA-2812, Fa. Byk-Gardner) mit dem Gerät BYK-mac (Fa. Byk-Gardner) ermittelt. Die Nagellacke wurden hierbei manuell mit einem Erichsen Film Applicator (System Wasag, Model 288, Fa. Erichsen) auf die Schwarz-Weiß-Deckungskarten aufgetragen (Naßfilmdicke 120 $\mu$m). Der Flopindex ist nach Alman folgendermaßen definiert (S. Schellenberger, M. Entenmann, A. Hennemann, P. Thometzek, Farbe und Lack, 04/2007, S.130):

$$\text{Flopindex} = 2{,}69 \cdot (L_{E1} - L_{E3})^{1,11} / L_{E2}^{0,86}$$

wobei $L_{E1}$ für die Helligkeit des glanznahen Messwinkels (E1 = 15° zum Glanzwinkel), $L_{E2}$ für die Helligkeit des Messwinkels zwischen glanznahem und glanzfernen Winkel (E2 = 45° zum Glanzwinkel) und $L_{E3}$ für die Helligkeit des glanzfernen Messwinkels (E3 = 110° zum Glanzwinkel) steht.

Die in Tabelle 2 aufgeführten Werte wurden auf dem schwarzen Untergrund der Schwarz-Weiß-Deckungskarte vermessen. In Tabelle 2 ist nur Phase A der Nagellacke beschrieben, als Phase B wurde, wie oben beschrieben, die kommerziell erhältliche Nagellackbasis International Lacquers Nailpolish & Care Base 359 (ad 100 Gew.-%) verwendet.

Tabelle 2: Helligkeit L* und Flopindex

| Beispiel/ Vergleichsbeispiel | Produktname | Gew-% | Mess-geometrie | L* | Flopindex |
|---|---|---|---|---|---|
| Vergleichsbeispiel 1 | Visionaire Bright Silver Sea | 2,00 | -15 | 139,5 | 23,5 |
| | | | 15 | 119,2 | |
| | | | 25 | 72,9 | |
| | | | 45 | 32,5 | |
| | | | 75 | 17,8 | |
| | | | 110 | 14,5 | |
| Beispiel 1 | Visionaire Bright Silver Sea | 2,00 | -15 | 97,7 | 7,1 |
| | | | 15 | 86,8 | |
| | | | 25 | 73,1 | |
| | Ceraflour 913 | 5,00 | 45 | 53,3 | |
| | | | 75 | 40,7 | |
| | | | 110 | 34,7 | |
| Beispiel 2 | Visionaire Bright Silver Sea | 2,00 | -15 | 99,7 | 7,1 |
| | | | 15 | 89,0 | |
| | | | 25 | 73,3 | |
| | Ceraflour 914 | 5,00 | 45 | 53,5 | |
| | | | 75 | 41,6 | |
| | | | 110 | 36,8 | |

(fortgesetzt)

| Beispiel/ Vergleichsbeispiel | Produktname | Gew-% | Mess-geometrie | L* | Flopindex |
|---|---|---|---|---|---|
| Vergleichsbeispiel 2 | Visionaire Bright Silver Sea | 2,00 | -15 | 99,2 | 5,7 |
| | | | 15 | 88,8 | |
| | | | 25 | 79,9 | |
| | Talk | 20,00 | 45 | 61,5 | |
| | | | 75 | 46,6 | |
| | | | 110 | 40,7 | |
| Vergleichsbeispiel 3 | Visionaire Sparkling Silver Sea | 2,00 | -15 | 130,6 | 26,2 |
| | | | 15 | 108,4 | |
| | | | 25 | 60,5 | |
| | | | 45 | 25,8 | |
| | | | 75 | 14,3 | |
| | | | 110 | 12,1 | |
| Beispiel 3 | Visionaire Sparkling Silver Sea | 2,00 | -15 | 110,0 | 7,5 |
| | | | 15 | 98,1 | |
| | | | 25 | 84,0 | |
| | Ceraflour 913 | 5,00 | 45 | 61,0 | |
| | | | 75 | 43,9 | |
| | | | 110 | 36,9 | |
| Beispiel 4 | Visionaire Sparkling Silver Sea | 2,00 | -15 | 85,7 | 3,8 |
| | | | 15 | 76,9 | |
| | | | 25 | 70,5 | |
| | Ceraflour 913 | 10,00 | 45 | 57,5 | |
| | | | 75 | 48,8 | |
| | | | 110 | 45,7 | |
| Beispiel 5 | Visionaire Sparkling Silver Sea | 2,00 | -15 | 84,3 | 1,3 |
| | | | 15 | 77,2 | |
| | | | 25 | 72,9 | |
| | Ceraflour 913 | 15,00 | 45 | 65,1 | |
| | | | 75 | 62,7 | |
| | | | 110 | 63,8 | |
| Beispiel 6 | Visionaire Sparkling Silver Sea | 2,00 | -15 | 85,3 | 0,7 |
| | | | 15 | 79,4 | |
| | | | 25 | 76.2 | |
| | Ceraflour 913 | 20,00 | 45 | 70,3 | |
| | | | 75 | 69,7 | |
| | | | 110 | 71,1 | |

(fortgesetzt)

| Beispiel/ Vergleichsbeispiel | Produktname | Gew-% | Mess-geometrie | L* | Flopindex |
|---|---|---|---|---|---|
| Beispiel 7 | Visionaire Sparkling Silver Sea | 2,00 | -15 | 109,2 | 8,1 |
| | | | 15 | 97,2 | |
| | | | 25 | 80,9 | |
| | Ceraflour 914 | 5,00 | 45 | 58,0 | |
| | | | 75 | 40,3 | |
| | | | 110 | 34,5 | |
| Beispiel 8 | Visionaire Sparkling Silver Sea | 2,00 | -15 | 83,8 | 2,6 |
| | | | 15 | 74,8 | |
| | | | 25 | 69,1 | |
| | Ceraflour 914 | 10,00 | 45 | 58,6 | |
| | | | 75 | 52,4 | |
| | | | 110 | 51,9 | |
| Beispiel 9 | Visionaire Sparkling Silver Sea | 2,00 | -15 | 86,5 | 1,7 |
| | | | 15 | 77,9 | |
| | | | 25 | 72,8 | |
| | Ceraflour 914 | 15,00 | 45 | 63,7 | |
| | | | 75 | 60,2 | |
| | | | 110 | 61,4 | |
| Beispiel 10 | Visionaire Sparkling Silver Sea | 5,00 | -15 | 94,6 | 1,8 |
| | | | 15 | 84,7 | |
| | | | 25 | 78,7 | |
| | Ceraflour 914 | 20,00 | 45 | 68,4 | |
| | | | 75 | 64,9 | |
| | | | 110 | 66,3 | |
| Vergleichsbeispiel 4 | Visionaire Sparkling Silver Sea | 2,00 | -15 | 144,0 | 21,9 |
| | | | 15 | 121,7 | |
| | | | 25 | 78,3 | |
| | Talk | 5,00 | 45 | 35,8 | |
| | | | 75 | 19,3 | |
| | | | 110 | 16,0 | |
| Vergleichsbeispiel 5 | Visionaire Sparkling Silver Sea | 2,00 | -15 | 134,5 | 15,8 |
| | | | 15 | 116,3 | |
| | | | 25 | 85,0 | |
| | Talk | 10,00 | 45 | 45,0 | |
| | | | 75 | 26,0 | |
| | | | 110 | 22,1 | |

(fortgesetzt)

| Beispiel/ Vergleichsbeispiel | Produktname | Gew-% | Mess-geometrie | L* | Flopindex |
|---|---|---|---|---|---|
| Vergleichsbeispiel 6 | Visionaire Sparkling Silver Sea | 2,00 | -15 | 121,2 | 10,8 |
| | | | 15 | 106,3 | |
| | | | 25 | 85,9 | |
| | Talk | 15,00 | 45 | 53,5 | |
| | | | 75 | 33,8 | |
| | | | 110 | 29,8 | |
| Vergleichsbeispiel 7 | Visionaire Sparkling Silver Sea | 2,00 | -15 | 110,2 | 6,9 |
| | | | 15 | 100,4 | |
| | | | 25 | 87,8 | |
| | Talk | 20,00 | 45 | 63,2 | |
| | | | 75 | 46,6 | |
| | | | 110 | 42,5 | |
| Vergleichsbeispiel 8 | Visionaire Sparkling Silver Sea | 1,00 | -15 | 112,5 | 25,8 |
| | | | 15 | 92,4 | |
| | | | 25 | 51,5 | |
| | Mirage Glamour Red | 1,00 | 45 | 21,5 | |
| | | | 75 | 12,0 | |
| | | | 110 | 9,8 | |
| Beispiel 16 | Visionaire Sparkling Silver Sea | 1,00 | -15 | 93,6 | 8,1 |
| | | | 15 | 81,3 | |
| | Mirage Glamour Red | 1,00 | 25 | 67,7 | |
| | | | 45 | 46,7 | |
| | Ceraflour 913 | 5,00 | 75 | 33,0 | |
| | | | 110 | 28,4 | |
| Beispiel 17 | Visionaire Sparkling Silver Sea | 1,00 | 15 | 94,9 | 8,2 |
| | | | 15 | 82,5 | |
| | Mirage Glamour Red | 1,00 | 25 | 67,0 | |
| | | | 45 | 46,2 | |
| | Ceraflour 914 | 5,00 | 75 | 32,6 | |
| | | | 110 | 29,2 | |
| Vergleichsbeispiel 9 | Visionaire Sparkling Silver Sea | 1,00 | -15 | 97,8 | 6,8 |
| | | | 15 | 86,7 | |
| | Mirage Glamour Red | 1,00 | 25 | 75,2 | |
| | | | 45 | 53,8 | |
| | Talk | 20,00 | 75 | 39,7 | |
| | | | 110 | 36,2 | |

(fortgesetzt)

| Beispiel/ Vergleichsbeispiel | Produktname | Gew-% | Mess-geometrie | L* | Flopindex |
|---|---|---|---|---|---|
| Vergleichsbeispiel 10 | Silverdream Moonlight 501L | 2,00 | -15 | 173,1 | 28,7 |
| | | | 15 | 149,9 | |
| | | | 25 | 86,6 | |
| | | | 45 | 35,0 | |
| | | | 75 | 22,0 | |
| | | | 110 | 17,3 | |
| Beispiel 18 | Silverdream Moonlight 501L | 2,00 | -15 | 113,9 | 5,3 |
| | | | 15 | 103,7 | |
| | | | 25 | 90,2 | |
| | Ceraflour 913 | 5,00 | 45 | 71,0 | |
| | | | 75 | 59,8 | |
| | | | 110 | 53,4 | |
| Beispiel 19 | Silverdream Moonlight 501L | 2,00 | -15 | 120,7 | 6,4 |
| | | | 15 | 110,5 | |
| | | | 25 | 90,3 | |
| | Ceraflour 914 | 5,00 | 45 | 68,0 | |
| | | | 75 | 57,3 | |
| | | | 110 | 53,0 | |
| Vergleichsbeispiel 11 | Silverdream Moonlight 501L | 2,00 | -15 | 115,6 | 5,3 |
| | | | 15 | 104,7 | |
| | | | 25 | 95,6 | |
| | Talk | 20,00 | 45 | 76,7 | |
| | | | 75 | 60,4 | |
| | | | 110 | 51,2 | |
| Vergleichsbeispiel 21 | SynCrystal Silver | 2,00 | -15 | 112,9 | 24,0 |
| | | | 15 | 95,7 | |
| | | | 25 | 54,5 | |
| | | | 45 | 24,2 | |
| | | | 75 | 14,2 | |
| | | | 110 | 11,0 | |
| Beispiel 38 | SynCrystal Silver | 2,00 | -15 | 85,0 | 5,9 |
| | | | 15 | 74,8 | |
| | | | 25 | 64,4 | |
| | Ceraflour 913 | 5,00 | 45 | 47,9 | |
| | | | 75 | 38,4 | |
| | | | 110 | 34,0 | |

(fortgesetzt)

| Beispiel/ Vergleichsbeispiel | Produktname | Gew-% | Mess-geometrie | L* | Flopindex |
|---|---|---|---|---|---|
| Beispiel 39 | SynCrystal Silver | 2,00 | -15 | 87,5 | 6,2 |
| | | | 15 | 76,9 | |
| | | | 25 | 64,2 | |
| | Ceraflour 914 | 5,00 | 45 | 47,3 | |
| | | | 75 | 38,6 | |
| | | | 110 | 34,9 | |
| Vergleichsbeispiel 22 | SynCrystal Silver | 2,00 | -15 | 90,5 | 5,1 |
| | | | 15 | 79,5 | |
| | | | 25 | 71,9 | |
| | Talk | 20,00 | 45 | 55,5 | |
| | | | 75 | 43,9 | |
| | | | 110 | 39,8 | |
| Vergleichsbeispiel 23 | SynCrystal Sparkling Silver | 2,00 | -15 | 91,1 | 24,9 |
| | | | 15 | 74,4 | |
| | | | 25 | 40,2 | |
| | | | 45 | 16,9 | |
| | | | 75 | 10,1 | |
| | | | 110 | 7,8 | |
| Beispiel 40 | SynCrystal Sparkling Silver | 2,00 | -15 | 82,9 | 7,5 |
| | | | 15 | 71,9 | |
| | | | 25 | 61,2 | |
| | Ceraflour 913 | 5,00 | 45 | 42,2 | |
| | | | 75 | 30,5 | |
| | | | 110 | 26,3 | |
| Beispiel 41 | SynCrystal Sparkling Silver | 2,00 | -15 | 84,4 | 7,7 |
| | | | 15 | 73,6 | |
| | | | 25 | 61,6 | |
| | Ceraflour 914 | 5,00 | 45 | 42,4 | |
| | | | 75 | 30,2 | |
| | | | 110 | 26,7 | |
| Vergleichsbeispiel 24 | SynCrystal Sparkling Silver | 2,00 | -15 | 88,8 | 6,7 |
| | | | 15 | 77,7 | |
| | | | 25 | 66,9 | |
| | Talk | 20,00 | 45 | 46,8 | |
| | | | 75 | 35,3 | |
| | | | 110 | 33,0 | |

(fortgesetzt)

| Beispiel/ Vergleichsbeispiel | Produktname | Gew-% | Mess-geometrie | L* | Flopindex |
|---|---|---|---|---|---|
| Vergleichsbeispiel 27 | Mirage Bright Blue | 2,00 | -15 | 71,8 | 22,3 |
| | | | 15 | 56,4 | |
| | | | 25 | 31,1 | |
| | | | 45 | 12,9 | |
| | | | 75 | 9,1 | |
| | | | 110 | 7,7 | |
| Beispiel 53 | Mirage Bright Blue | 2,00 | -15 | 60,0 | 6,4 |
| | | | 15 | 46,0 | |
| | | | 25 | 36,8 | |
| | Ceraflour 913 | 5,00 | 45 | 25,2 | |
| | | | 75 | 21,0 | |
| | | | 110 | 19,6 | |
| Beispiel 54 | Mirage Bright Blue | 2,00 | -15 | 60,5 | 6,4 |
| | | | 15 | 46,8 | |
| | | | 25 | 37,1 | |
| | Ceraflour 914 | 5,00 | 45 | 25,4 | |
| | | | 75 | 21,5 | |
| | | | 110 | 20,2 | |
| Vergleichsbeispiel 28 | Mirage Bright Blue | 2,00 | -15 | 66,1 | 4,4 |
| | | | 15 | 50,6 | |
| | | | 25 | 44,7 | |
| | Talk | 20,00 | 45 | 33,1 | |
| | | | 75 | 27,2 | |
| | | | 110 | 27,0 | |

Je größer der Zahlenwert des Flopindexes ist, desto stärker kommt der Hell-/ Dunkelflop zum Ausdruck. Die erfindungsgemäßen Nagellacke wiesen in der Applikationen einen deutlich geringeren Flopindex auf. Bei einem Vergleich der Flopindexwerte der Applikationen eines mit dem Metalleffektpigment Visionaire Sparkling Silver Sea pigmentierten Nagellacks ohne mikronisiertes Wachs und in Gegenwart wenigstens eines mikronisierten Wachses, ist eine Verringerung des Flopindex von 26,2 (Vergleichsbeispiel 3) um ca. 18 Einheiten auf 7,5 (Beispiel 3) bzw. 8,1 (Beispiel 7) erkennbar.

Würde anstelle des wenigstens einen mikronisierten Wachses zur Erzielung eines schwach ausgeprägten Hell-/ Dunkelflops (niedriger Zahlenwert des Flopindex) im applizierten Nagellack ein handelsübliches Mattierungsmittel wie beispielsweise Talk eingesetzt, so müsste zur Erzielung einer vergleichbaren optischen Effekts eine deutlich größere Menge an Mattierungsmittel zugefügt werden. Während die Anwesenheit von 5 Gew.-% wenigstens eines mikronisierten Wachses bereits eine deutliche Verringerung des Flopindex bewirkte, war in Gegenwart von 5 Gew.-% Talk immer noch ein deutlicher Hell-/ Dunkelflop mit entsprechend hohem Wert des Flopindex von 21,9 zu beobachten (Vergleichbeispiel 4). Der Zusatz von 10 Gew.-% Talk verringerte diesen Wert auf 15,8 (Vergleichsbeispiel 5), aber erst durch Zugabe von 15 Gew.% Talk konnte ein Flopindex von 10,8 erreicht werden (Vergleichsbeispiel 6). Um also einen vergleichbaren optischen Effekt wie durch Zugabe von 5 Gew.% mikronisiertem Wachs zu erzielen, mussten aber wenigstens 15 Gew.-% Talk eingesetzt werden (Vergleichsbeispiel 6). Bei Zugabe von 20 Gew.-% Talk ließ sich schließlich ein Flopindex des applizierten Nagellacks von 6,9 messen (Vergleichsbeispiel 7).

IIIb Glanzmessungen

Die Glanzmessungen wurden anhand von Nagellackapplikationen auf Schwarz-Weiß-Deckungskarten (Chart PA-2812, Fa. Byk-Gardner) mit dem Gerät micro-TRI-gloss µ (Fa. Byk-Gardner) gemäß Herstellerangaben bei einer Messgeometrie von 20° und 60°, bezogen auf die Vertikale, jeweils auf weißem und schwarzem Untergrund durchgeführt. Die Nagellacke wurden hierbei manuell mit einem Erichsen Film Applicator (System Wasag, Model 288, Fa. Erichsen) auf die Schwarz-Weiß-Deckungskarten aufgetragen (Naßfilmdicke 120 µm). Eine Messgeometrie von 60° ist für den sogenannten "Mittelglanz" im Bereich von 10 bis 70 Glanzpunkten geeignet, wobei ein höherer Zahlenwert bei den Glanzpunkten einem höheren Glanz entspricht. Eine Messgeometrie von 20° ist für den sogenannten "Hochglanz" geeignet, d.h. liegt der Glanzwert bei der Messgeometrie von 60° bei über 70 Glanzpunkten, so wird bei einer Messgeometrie von 20° gemessen (Byk-Gardner, Katalog "Qualitätskontrolle für Lacke und Kunststoffe" 2011/2012, S. 16). Die nachstehend in Tabelle 3 aufgeführten Glanzwerte stellen Mittelwerte aus jeweils fünf Einzelmessungen dar. In Tabelle 3 wird der Einfachheit halber nur Phase A der Nagellacke aufgeführt, als Phase B wurde, wie oben beschrieben, die kommerziell erhältliche Nagellackbasis International Lacquers Nailpolish & Care Base 359 (ad 100 Gew.-%) verwendet.

Tabelle 3: Glanzwerte von Nagellackapplikationen bei 20° und 60°

| Beispiel/ Vergleichsbeispiel | Produktname | Gew.-% | Glanz (weiß) | | Glanz (schwarz) | |
|---|---|---|---|---|---|---|
| | | | 20° | 60° | 20° | 60° |
| Vergleichsbeispiel 3 | Visionaire Sparkling Silver Sea | 2,00 | 42,0 | 92,1 | 40,0 | 89,3 |
| Beispiel 3 | Visionaire Sparkling Silver Sea | 2,00 | 2,3 | 5,5 | 2,1 | 5,7 |
| | Ceraflour 913 | 5,00 | | | | |
| Beispiel 4 | Visionaire Sparkling Silver Sea | 2,00 | 1,4 | 2,8 | 0,8 | 2,1 |
| | Ceraflour 913 | 10,00 | | | | |
| Beispiel 5 | Visionaire Sparkling Silver Sea | 2,00 | 1,2 | 2,3 | 0,8 | 1,9 |
| | Ceraflour 913 | 15,00 | | | | |
| Beispiel 6 | Visionaire Sparkling Silver Sea | 2,00 | 1,2 | 2,3 | 0,9 | 2,0 |
| | Ceraflour 913 | 20,00 | | | | |
| Beispiel 7 | Visionaire Sparkling Silver Sea | 2,00 | 3,0 | 7,0 | 3,2 | 8,1 |
| | Ceraflour 914 | 5,00 | | | | |
| Beispiel 8 | Visionaire Sparkling Silver Sea | 2,00 | 1,4 | 2,7 | 0,8 | 2,0 |
| | Ceraflour 914 | 10,00 | | | | |
| Beispiel 9 | Visionaire Sparkling Silver Sea | 2,00 | 1,3 | 2,6 | 0,8 | 2,1 |
| | Ceraflour 914 | 15,00 | | | | |
| Beispiel 10 | Visionaire Sparkling Silver Sea | 2,00 | 1,2 | 2,9 | 1,0 | 2,7 |
| | Ceraflour 914 | 20,00 | | | | |
| Vergleichsbeispiel 4 | Visionaire Sparkling Silver Sea | 2,00 | 9,9 | 26,6 | 9,8 | 27,9 |
| | Talk | 5,00 | | | | |
| Vergleichsbeispiel 5 | Visionaire Sparkling Silver Sea | 2,00 | 4,5 | 11,6 | 4,2 | 11,2 |
| | Talk | 10,00 | | | | |
| Vergleichsbeispiel 6 | Visionaire Sparkling Silver Sea | 2,00 | 2,9 | 7,0 | 2,4 | 6,2 |
| | Talk | 15,00 | | | | |
| Vergleichsbeispiel 7 | Visionaire Sparkling Silver Sea | 2,00 | 2,1 | 4,8 | 1,8 | 4,5 |
| | Talk | 20,00 | | | | |
| Vergleichsbeispiel 12 | Stapa WM Iron VP 401040 | 2,00 | 16,4 | 58,9 | 17,1 | 60,2 |

(fortgesetzt)

| Beispiel/ Vergleichsbeispiel | Produktname | Gew.-% | Glanz (weiß) | | Glanz (schwarz) | |
|---|---|---|---|---|---|---|
| | | | 20° | 60° | 20° | 60° |
| Beispiel 20 | Stapa WM Iron VP 401040 | 2,00 | 0,9 | 2,7 | 1,2 | 4,0 |
| | Ceraflour 913 | 5,00 | | | | |
| Beispiel 24 | Stapa WM Iron VP 401040 | 2,00 | 1,1 | 3,4 | 1,2 | 4,0 |
| | Ceraflour 914 | 5,00 | | | | |
| Beispiel 25 | Stapa WM Iron VP 401040 | 2,00 | 0,5 | 1,0 | 0,4 | 0,9 |
| | Ceraflour 914 | 10,00 | | | | |
| Beispiel 26 | Stapa WM Iron VP 401040 | 2,00 | 0,4 | 0,8 | 0,2 | 0,6 |
| | Ceraflour 914 | 15,00 | | | | |
| Beispiel 27 | Stapa WM Iron VP 401040 | 2,00 | 0,4 | 0,9 | 0,2 | 0,8 |
| | Ceraflour 914 | 20,00 | | | | |
| Vergleichsbeispiel 13 | Stapa WM Iron VP 401040 | 2,00 | 3,5 | 14,7 | 3,7 | 16,1 |
| | Talk | 5,00 | | | | |
| Vergleichsbeispiel 14 | Stapa WM Iron VP 401040 | 2,00 | 1,6 | 6,1 | 1,6 | 6,3 |
| | Talk | 10,00 | | | | |
| Vergleichsbeispiel 15 | Stapa WM Iron VP 401040 | 2,00 | 1,0 | 3,6 | 1,0 | 3,6 |
| | Talk | 15,00 | | | | |
| Vergleichsbeispiel 16 | Stapa WM Iron VP 401040 | 2,00 | 0,7 | 2,3 | 0,7 | 2,3 |
| | Talk | 20,00 | | | | |

IIIc Effektmessungen

Effektmessungen zur Bestimmung des optischen Einflusses des wenigstens einen mikronisierten Wachses wurden anhand von Nagellackapplikationen auf Schwarz-Weiß-Deckungskarten (Chart PA-2812, Fa. Byk-Gardner) mit einem BYK-mac (Fa. Byk-Gardner) durchgeführt. Die Nagellacke wurden hierbei manuell mit einem Erichsen Film Applicator (System Wasag, Model 288, Fa. Erichsen) auf die Schwarz-Weiß-Deckungskarten aufgetragen (Naßfilmdicke 120 $\mu$m). Die beispielhaft ausgewählten Nagellacke wiesen eine unterschiedliche Pigmentierungshöhe an Effektpigment, unterschiedliche Mengen an mikronisierten Wachsen sowie verschiedene mikronisierte Wachse auf. Als Referenz wurde jeweils der mit dem entsprechenden Effektpigment pigmentierte Nagellack ohne Zusatz eines mikronisierten Wachses bzw. durch Zusatz von Talk vermessen.

Zur Simulation von Effektänderungen bei direkter Beleuchtung wird der Glitzereffekt mit dem BYK-mac unter Verwendung einer hochauflösenden CCD-Kamera untersucht. Der Glitzereffekt, verursacht durch das Reflexionsvermögen der einzelnen Effektpigmente, wird nur bei direkter Sonneneinstrahlung wahrgenommen und verändert sich in Abhängigkeit des Beleuchtungswinkels. Aus diesem Grund wird die Probe bei dem Byk-mac mit sehr hellen LEDs unter drei verschiedenen Winkeln (15°/ 45°/ 75°) beleuchtet. Mit der CCD-Kamera wird dabei senkrecht zur Oberfläche jeweils ein Bild aufgenommen. Die Bilder werden mit Hilfe von Bildverarbeitungsalgorithmen analysiert, wobei das Histogramm der Helligkeitsstufen als Basis für die Berechnung der Glitzerparameter verwendet wird. Um eine bessere Differenzierung zu gewährleisten, wurde der Glitzereffekt durch ein zweidimensionales System beschrieben, der Glitzerfläche S_a und der Glitzerintensität SAlternativ wurden genannte Angaben durch den eindimensionalen Wert, den Glitzergrad S_G zusammengefasst. Die entsprechenden Messwerte sind in Tabelle 4 zusammengefasst. Auch hier ist nur Phase A erwähnt, als Phase B wurde die kommerziell erhältliche Nagellackbasis International Lacquers Nailpolish & Care Base 359 (ad 100 Gew.-%) verwendet.

Tabelle 4: Glitzergrad S_G, Glitzerintensität S_i und Glitzerfläche S_a

| Beispiel/ Vergleichsbeispiel | Produktname | Gew-% | Messgeometrie | S_G | S_i | S_a |
|---|---|---|---|---|---|---|
| Vergleichsbeispiel 1 | Visionaire Bright Silver Sea | 2,00 | 15 | 6,5 | 16,4 | 26.2 |
| | | | 45 | 4,7 | 12,0 | 20,5 |
| | | | 75 | 3,9 | 13,0 | 13,8 |
| Beispiel 1 | Visionaire Bright Silver Sea | 2,00 | 15 | 6,5 | 14,4 | 30,5 |
| | Ceraflour 913 | 5,00 | 45 | 7,5 | 16,9 | 33,2 |
| | | | 75 | 9,3 | 28,0 | 29,9 |
| Beispiel 2 | Visionaire Bright Silver Sea | 2,00 | 15 | 7,5 | 17,8 | 31,7 |
| | Ceraflour 914 | 5,00 | 45 | 8,5 | 22,2 | 31,7 |
| | | | 75 | 10,1 | 32,2 | 30,1 |
| Vergleichsbeispiel 2 | Visionaire Bright Silver Sea | 2,00 | 15 | 4,6 | 10,1 | 24,1 |
| | Talk | 20,00 | 45 | 5,4 | 11,5 | 27,4 |
| | | | 75 | 6,1 | 15,5 | 25,5 |
| Vergleichsbeispiel 3 | Visionaire Sparkling Silver Sea | 2,00 | 15 | 9,1 | 25,1 | 31,8 |
| | | | 45 | 4,1 | 9,9 | 19,6 |
| | | | 75 | 3,4 | 13,3 | 10,8 |
| Beispiel 3 | Visionaire Sparkling Silver Sea | 2,00 | 15 | 8,8 | 21,6 | 34,6 |
| | Ceraflour 913 | 5,00 | 45 | 9,7 | 29,2 | 30,6 |
| | | | 75 | 10,5 | 40,8 | 25,4 |
| Beispiel 4 | Visionaire Sparkling Silver Sea | 2,00 | 15 | 7,4 | 20,0 | 27,5 |
| | Ceraflour 913 | 10,00 | 45 | 7,8 | 21,8 | 28,0 |
| | | | 75 | 8,1 | 26,3 | 24.6 |
| Beispiel 5 | Visionaire Sparkling Silver Sea | 2,00 | 15 | 7,4 | 20,0 | 27,3 |
| | Ceraflour 913 | 15,00 | 45 | 6,6 | 17,9 | 25,1 |
| | | | 75 | 6,1 | 21,0 | 18,6 |
| Beispiel 6 | Visionaire Sparkling Silver Sea | 2,00 | 15 | 7,3 | 20,1 | 26,8 |
| | Ceraflour 913 | 20,00 | 45 | 6,2 | 20,2 | 19,6 |
| | | | 75 | 4,9 | 20,0 | 13.1 |
| Beispiel 7 | Visionaire Sparkling Silver Sea | 2,00 | 15 | 11,0 | 33,8 | 33,6 |
| | Ceraflour 914 | 5,00 | 45 | 12,0 | 46,7 | 28,7 |
| | | | 75 | 12,2 | 58,3 | 23,6 |
| Beispiel 8 | Visionaire Sparkling Silver Sea | 2,00 | 15 | 9,7 | 31,1 | 29,0 |
| | Ceraflour 914 | 10,00 | 45 | 9,6 | 33,3 | 26,2 |
| | | | 75 | 8,9 | 36,5 | 20,9 |
| Beispiel 9 | Visionaire Sparkling Silver Sea | 2,00 | 15 | 10,3 | 34,8 | 28,7 |
| | Ceraflour 914 | 15,00 | 45 | 9,9 | 40,3 | 23,2 |
| | | | 75 | 7,7 | 33,6 | 17,5 |

(fortgesetzt)

| Beispiel/ Vergleichsbeispiel | Produktname | Gew-% | Messgeometrie | S_G | S_i | S_a |
|---|---|---|---|---|---|---|
| Beispiel 10 | Visionaire Sparkling Silver Sea | 2,00 | 15 | 11,8 | 43,6 | 29,6 |
| | Ceraflour 914 | 20,00 | 45 | 10,8 | 52,0 | 21,2 |
| | | | 75 | 7,1 | 37,2 | 13,5 |
| Vergleichsbeispiel 4 | Visionaire Sparkling Silver Sea | 2,00 | 15 | 8,6 | 21,6 | 33,3 |
| | Talk | 5,00 | 45 | 7,3 | 20,3 | 26,4 |
| | | | 75 | 5,4 | 19,5 | 16,3 |
| Vergleichsbeispiel 5 | Visionaire Sparkling Silver Sea | 2,00 | 15 | 7,6 | 18,5 | 31,2 |
| | Talk | 10,00 | 45 | 7,3 | 18,6 | 29,1 |
| | | | 75 | 5,5 | 15,9 | 20,6 |
| Vergleichsbeispiel 6 | Visionaire Sparkling Silver Sea | 2,00 | 15 | 6,3 | 14,2 | 28,9 |
| | Talk | 15,00 | 45 | 6,5 | 15,1 | 28,8 |
| | | | 75 | 5,8 | 15,8 | 22,4 |
| Vergleichsbeispiel 7 | Visionaire Sparkling Silver Sea | 2,00 | 15 | 7,0 | 15,8 | 31,0 |
| | Talk | 20,00 | 45 | 6,5 | 14,7 | 29,8 |
| | | | 75 | 5,7 | 13,9 | 24,8 |
| Vergleichsbeispiel 8 | Visionaire Sparkling Silver Sea | 1,00 | 15 | 10,0 | 28,9 | 32,8 |
| | Mirage Glamour Red | 1,00 | 45 | 5,0 | 15,4 | 18,1 |
| | | | 75 | 4,0 | 17,3 | 10,9 |
| Beispiel 16 | Visionaire Sparkling Silver Sea | 1,00 | 15 | 9,4 | 23,8 | 35,3 |
| | Mirage Glamour Red | 1,00 | 45 | 9,9 | 29,8 | 31,5 |
| | Ceraflour 913 | 5,00 | 75 | 10,3 | 42,5 | 23,7 |
| Beispiel 17 | Visionaire Sparkling Silver Sea | 1,00 | 15 | 11,0 | 32,9 | 34,7 |
| | Mirage Glamour Red | 1,00 | 45 | 11,7 | 44,7 | 28,3 |
| | Ceraflour 914 | 5,00 | 75 | 11,2 | 60,2 | 19,4 |
| Vergleichsbeispiel 9 | Visionaire Sparkling Silver Sea | 1,00 | 15 | 6,3 | 13,8 | 29,8 |
| | Mirage Glamour Red | 1,00 | 45 | 5,7 | 16,1 | 28,4 |
| | Talk | 20,00 | 75 | 5,5 | 14,7 | 21,7 |
| Vergleichsbeispiel 10 | Silverdream Moonlight 50IL | 2,00 | 15 | 1,6 | 5,5 | 8,8 |
| | | | 45 | 3,1 | 7,0 | 17,6 |
| | | | 75 | 3,6 | 13,9 | 11,3 |
| Beispiel 18 | Silverdream Moonlight 50IL | 2,00 | 15 | 3,2 | 6,0 | 21,9 |
| | Ceraflour 913 | 5,00 | 45 | 4,0 | 6,7 | 27,7 |
| | | | 75 | 6,4 | 12,3 | 34,6 |
| Beispiel 19 | Silverdream Moonlight 50IL | 2,00 | 15 | 4,1 | 8,0 | 24,6 |
| | Ceraflour 914 | 5,00 | 45 | 4,8 | 8,2 | 31,0 |
| | | | 75 | 7,3 | 14,8 | 36,2 |

(fortgesetzt)

| Beispiel/ Vergleichsbeispiel | Produktname | Gew-% | Messgeometrie | S_G | S_i | S_a |
|---|---|---|---|---|---|---|
| Vergleichsbeispiel 11 | Silverdream Moonlight 50IL | 2,00 | 15 | 2,4 | 5,0 | 16,8 |
| | Talk | 20,00 | 45 | 3,6 | 6,5 | 24,5 |
| | | | 75 | 5,6 | 11,3 | 29,8 |
| Vergleichsbeispiel 21 | SynCrystal Silver | 2,00 | 15 | 3,8 | 8,4 | 20,5 |
| | | | 45 | 1,2 | 3,7 | 8,7 |
| | | | 75 | 1,1 | 6,5 | 4,7 |
| Beispiel 38 | SynCrystal Silver | 2,00 | 15 | 5,0 | 9,3 | 30,0 |
| | Ceraflour 913 | 5,00 | 45 | 6,1 | 12,1 | 32,0 |
| | | | 75 | 7,4 | 19,0 | 29,0 |
| Beispiel 39 | SynCrystal Silver | 2,00 | 15 | 5,9 | 11,5 | 32,0 |
| | Ceraflour 914 | 5,00 | 45 | 7,2 | 16,1 | 32,1 |
| | | | 75 | 8,2 | 24,2 | 27,5 |
| Vergleichsbeispiel 22 | SynCrystal Silver | 2,00 | 15 | 3,8 | 7,5 | 23,2 |
| | Talk | 20,00 | 45 | 4,2 | 8,2 | 24,9 |
| | | | 75 | 4,6 | 10,8 | 22,0 |
| Vergleichsbeispiel 23 | SynCrystal Sparkling Silver | 2,00 | 15 | 6,5 | 16,7 | 26,4 |
| | | | 45 | 2,2 | 5,5 | 13,2 |
| | | | 75 | 1,9 | 9,1 | 6,5 |
| Beispiel 40 | SynCrystal Sparkling Silver | 2,00 | 15 | 8,2 | 19,0 | 34,5 |
| | Ceraflour 913 | 5,00 | 45 | 8,3 | 23,0 | 29,2 |
| | | | 75 | 8,4 | 28,6 | 23,9 |
| Beispiel 41 | SynCrystal Sparkling Silver | 2,00 | 15 | 10,0 | 27,8 | 34,2 |
| | Ceraflour 914 | 5,00 | 45 | 9,7 | 30,7 | 29,0 |
| | | | 75 | 9,1 | 38,8 | 20,5 |
| Vergleichsbeispiel 24 | SynCrystal Sparkling Silver | 2,00 | 15 | 5,8 | 12,8 | 27,9 |
| | Talk | 20,00 | 45 | 5,1 | 12,2 | 23,6 |
| | | | 75 | 3,9 | 10,6 | 16,8 |
| Vergleichsbeispiel 27 | Mirage Bright Blue | 2,00 | 15 | 6,8 | 16,6 | 28,6 |
| | | | 45 | 2,7 | 4,1 | 24,2 |
| | | | 75 | 1,8 | 5,4 | 10, 1 |
| Beispiel 53 | Mirage Bright Blue | 2,00 | 15 | 5,5 | 12,3 | 26,5 |
| | Ceraflour 913 | 5,00 | 45 | 5,7 | 15,3 | 22,5 |
| | | | 75 | 4,7 | 14,3 | 17,3 |
| Beispiel 54 | Mirage Bright Blue | 2,00 | 15 | 6,7 | 18,5 | 24,7 |
| | Ceraflour 914 | 5,00 | 45 | 6,2 | 18,8 | 21,2 |
| | | | 75 | 5,2 | 16,5 | 17,9 |

(fortgesetzt)

| Beispiel/ Vergleichsbeispiel | Produktname | Gew-% | Messgeometrie | S_G | S_i | S_a |
|---|---|---|---|---|---|---|
| Vergleichsbeispiel 28 | Mirage Bright Blue | 2,00 | 15 | 3,4 | 6,5 | 22,5 |
| | Talk | 20,00 | 45 | 2,8 | 5,3 | 19,6 |
| | | | 75 | 2,2 | 5,5 | 13,6 |

Entscheidend für den optischen Eindruck ist der eindimensionale Glitzergrad S_G. Je höher der Zahlenwert von S_G, desto höher ist der auch vom Auge wahrnehmbare Glitzereffekt. In einer zweidimensionalen Darstellung kann der Glitzergrad S_G in die Komponenten Glitzerintensität S_i und Glitzerfläche S_a aufgeteilt werden. Da beide Komponenten einen maßgeblichen Einfluss auf den Glitzergrad S_G haben, kann es vorkommen, dass ein Effektpigment in den Messgeometrien 15°, 45° und 75° nahezu den gleichen Glitzergrad S_G aufweist, obwohl die Zahlenwerte von S_a und S_G in den betrachteten Messgeometrien deutlich erhöht bzw. erniedrigt sind. So wies z.B. eine Nagellackapplikation von 2 Gew.% Visionaire Sparkling Silver Sea und 5 Gew.-% Ceraflour 913 (Beispiel 3) bei 15° Messgeometrie einen Glitzergrad S_G von 8,8 auf, wobei die Glitzerintensität 21,6 und die Glitzerfläche 34,6 betrug. Bei 75° wurde ein Glitzergrad S_G Wert von 10,5 ermittelt, d.h. der wahrnehmbare Glitzereffekt hatte sich wenig verändert. Dennoch veränderten sich die Komponenten Glitzerintensität S und Glitzerfläche S_a signifikanz- die Glitzerintensität S_i stieg auf 41, die Glitzerfläche S_a sank auf 25. Die reduzierte Glitzerfläche S_a wurde also durch die deutlich gestiegene Glitzerintensität vollständig kompensiert, so dass vom Betrachter nur eine unwesentliche Änderung des Glitzereffekts wahrgenommen wurde.

**IV Charakterisierung der optischen und haptischen Effekte der erfindungsgemäßen Nagellacke**

IVa Rasterelektronenmikroskopische Aufnahmen der Nagellacke

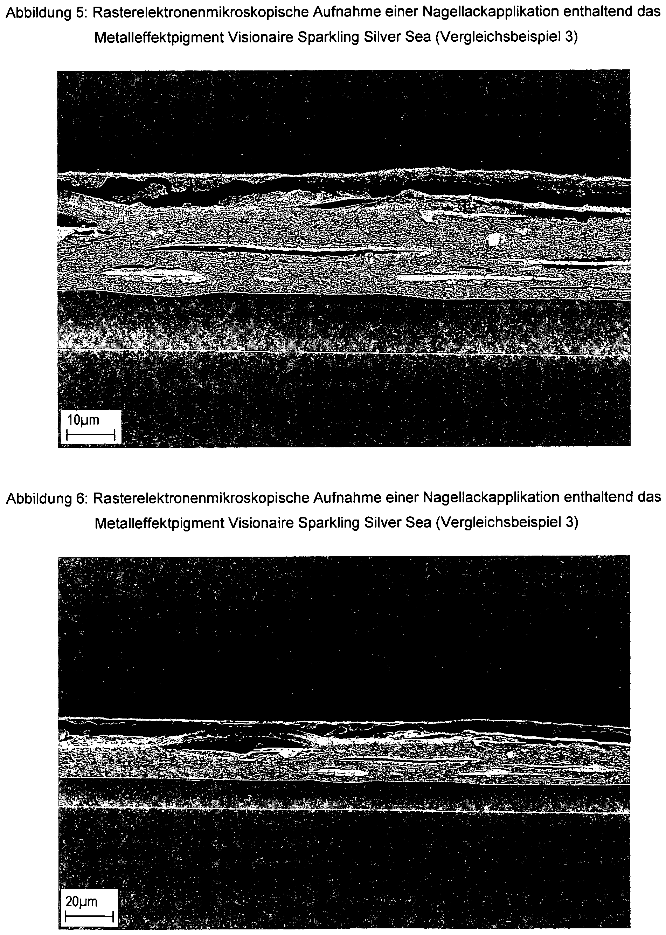

Die rasterelektronenmikroskopischen Aufnahmen (Abbildungen 5 bis 9) wurden anhand von Querschliffen der erfindungsgemäßen Nagellacke mit dem Rasterelektronenmikroskop Supra 35 (Fa. Zeiss) erhalten.

IVb Lichtmikroskopische Tiefenprofile der Nagellacke

Die Tiefenprofile wurden mit Hilfe von lichtmikroskopischen Aufnahmen von Nagellackapplikationen auf Schwarz-Weiß-Deckungskarten (Chart PA-2812, Fa. Byk-Gardner) mit dem Mikroskop Axio Imager.Z1 m (Fa. Zeiss) erstellt. Die Nagellacke wurden hierbei manuell mit einem Erichsen Film Applicator (System Wasag, Model 288, Fa. Erichsen) auf die Schwarz-Weiß-Deckungskarten aufgetragen (Naßfilmdicke 120 $\mu$m).
Beispielhaft wurden Nagellacke vermessen, welche mit einem Effektpigment, ohne mikronisiertes Wachs, unterschiedlichen mikronisierten Wachsen sowie mit Talk versehen waren (Tabelle 5).

Tabelle 5: Zusammensetzung der Nagellacke (Phase A) für mikroskopische Tiefenprofile, Phase B entspricht der oben beschriebenen

| Beispiel/ Vergleichsbeispiel | Produktname | Gew.% |
|---|---|---|
| Vergleichsbeispiel 3 | Visionaire Sparkling Silver Sea | 2,00 |
| Beispiel 3 | Visionaire Sparkling Silver Sea | 2,00 |
| | Ceraflour 913 | 5,00 |
| Beispiel 7 | Visionaire Sparkling Silver Sea | 2,00 |
| | Ceraflour 914 | 5,00 |
| Vergleichsbeispiel 7 | Visionaire Sparkling Silver Sea | 2,00 |
| | Talk | 20,00 |

Von den Nagellackapplikation wurden über einen Höhenbereich von ca. 30 $\mu$m senkrecht zur Schwarz-Weiß-Deckungskarte jeweils zwei Mikroskopbilder pro Mikrometer aufgenommen, welche anschließend mit Hilfe der Bildverarbeitungssoftware AxioVision 4.6.3. zu einem einzigen Mikroskopbild (Abbildungen 10 bis 13) übereinandergelegt und gleichzeitig zu einem Topographiebild verrechnet wurden. Aus diesem Topographiebild wurde nach DIN EN ISO 4287:2010-07 die Oberflächenkenngröße "Rauwert" Rq berechnet. Zum Vergleich wurde ein identisch pigmentierter Nagellack ohne den Zusatz von mikronisiertem Wachs herangezogen. Die Rauwerte der in Tabelle 5 beschriebenen Nagellacke sind in Abbildung 14 dargestellt. Ein Vergleich der Rauwerte belegte den haptisch spürbaren Einfluss eines mikronisierten Wachses. Ein Nagellack, welcher ausschließlich ein Effektpigment enthält, zeichnete sich durch seine vergleichsweise glatte Oberfläche aus, was sich auch im zugehörigen Topographiebild und entsprechend niedrigem Rauwert widerspiegelte. Die Zugabe von wenigstens einem mikronisierten Wachs wirkte sich deutlich auf das Topographiebild aus, die Berg- und Talstruktur wurde ausgeprägter, die zugehörigen Rauwerte stiegen im Vergleich zu einem Nagellack ohne mikronisiertes Wachs sehr stark an. Auch haptisch war dieser Einfluss zu spüren. Ein vergleichbarer haptischer Effekt und vergleichbare Rauwerte waren erst bei deutlich erhöhtem Talkgehalt zu beobachten.

**Patentansprüche**

1. Nagellack, umfassend wenigstens ein Farbmittel und wenigstens ein mikronisiertes Wachs,
   **dadurch gekennzeichnet,**
   **dass** das wenigstens eine Farbmittel ein plättchenförmiges Effektpigment ist und wobei der Nagellack das wenigstens eine mikronisierte Wachs in einer Menge aus einem Bereich von 0,1 bis 25 Gew.-%, bezogen auf das Gesamtgewicht des Nagellacks, enthält und das wenigstens eine mikronisierte Wachs eine mittlere Teilchengröße $D_{50}$ aus einem Bereich von 2,5 bis 91 $\mu$m aufweist.

2. Nagellack nach Anspruch 1,
   **dadurch gekennzeichnet,**
   **dass** das Effektpigment ein Metalleffektpigment und/ oder ein Perlglanzpigment ist.

3. Nagellack nach einem der vorstehenden Ansprüche,
   **dadurch gekennzeichnet,**
   **dass** das wenigstens eine mikronisierte Wachs ein natürliches und/ oder synthetisches Wachs umfasst.

4. Nagellack nach einem der vorstehenden Ansprüche,
   **dadurch gekennzeichnet,**
   **dass** das wenigstens eine mikronisierte Wachs ein synthetisches ungefärbtes Wachs ist.

5. Verfahren zur Herstellung eines Nagellacks nach einem der vorstehenden Ansprüche,
   **dadurch gekennzeichnet,**
   **dass** das Verfahren wenigstens folgenden Schritt umfasst:

   Vermengen einer Nagellackbasis, wenigstens eines mikronisierten Wachses und wenigstens eines Farbmittels unter Bereitstellung eines Nagellacks.

6. Gegenstand,
   **dadurch gekennzeichnet,**
   **dass** der Gegenstand einen einschichtig aufgebrachten Nagellack nach einem der Ansprüche 1 bis 4 aufweist, wobei der Gegenstand ein künstlicher Fingernagel ist.

**Claims**

1. Nail varnish, comprising at least one colourant and at least one micronized wax,
   **characterised in that**
   the at least one colourant is a platelet-shaped effect pigment and the nail varnish contains the at least one micronized wax in a quantity within a range of from 0.1 to 25 % by weight, based on the total weight of the nail varnish, and the at least one micronized wax has an average particle size $D_{50}$ within a range of from 2.5 to 91 $\mu$m.

2. Nail varnish as claimed in claim 1,
   **characterised in that**
   the effect pigment is a metal effect pigment and/ or a pearlescent pigment.

3. Nail varnish as claimed in one of the preceding claims, **characterised in that**

the at least one micronized wax comprises a natural and/ or synthetic wax.

4.  Nail varnish as claimed in one of the preceding claims, **characterised in that**
    the at least one micronized wax is a synthetic, uncoloured wax.

5.  Method of producing a nail varnish as claimed in one of the preceding claims,
    **characterised in that**
    the method comprises at least the following step:

    mixing a nail varnish base, at least one micronized wax and at least one colourant to obtain a nail varnish.

6.  Article,
    **characterised in that**
    the article comprises a nail varnish as claimed in one of claims 1 to 4 applied in a single layer and the article is an artificial finger nail.


**Revendications**

1.  Vernis à ongles, comprenant au moins un colorant et au moins une cire micronisée, **caractérisé en ce que** le ou les colorants sont des pigments lamellaires à effets, et le vernis à ongles contenant la ou les cires micronisées en une quantité comprise dans la plage de 0,1 à 25 % en poids par rapport au poids total du vernis à ongles, et la ou les cires micronisées présentent une granulométrie moyenne $D_{50}$ comprise dans la plage de 2,5 à 91 $\mu$m.

2.  Vernis à ongles selon la revendication 1, **caractérisé en ce que** le pigment à effets est un pigment à effets métallisés et/ou un pigment nacré.

3.  Vernis à ongles selon l'une des revendications précédentes, **caractérisé en ce que** la ou les cires micronisées sont des cires naturelles et/ou synthétiques.

4.  Vernis à ongles selon l'une des revendications précédentes, **caractérisé en ce que** la ou les cires micronisées sont des cires synthétiques non colorées.

5.  Procédé de fabrication d'un vernis à ongles selon l'une des revendications précédentes, **caractérisé en ce que** le procédé comprend au moins l'étape suivante : mélange d'une base pour vernis à ongles, d'au moins une cire micronisée et d'au moins un colorant, avec mise à disposition d'un vernis à ongles.

6.  Objet, **caractérisé en ce que** l'objet est un vernis à ongles appliqué en une couche selon l'une des revendications 1 à 4, l'objet étant un ongle artificiel.

Abbildung 1: Byk mac Effekt-Meßgeometrien (Byk-Gardner, Katalog "Qualitätskontrolle für Lacke und Kunststoffe" 2011/2012, S. 97)

**CCD-Chip**

45°

15°
Sparkle

15°

45°
Sparkle

45°

75°
Sparkle

45°

Graininess

75°

Abbildung 2: Glitzerfläche S_a und Glitzerintensität S_i einer Nagellackapplikation enthaltend das Metalleffektpigment Visionaire Sparkling Silver Sea ohne Zusatz von mikronisiertem Wachs (Vergleichsbeispiel 3), in Gegenwart von Ceraflour 913 (Beispiel 3), Ceraflour 914 (Beispiel 7) und Talk (Vergleichsbeispiel 7) bei einer Messgeometrie von 15° (vermessen auf schwarzem Untergrund einer Schwarz-Weiß-Deckungskarte)

Abbildung 3: Glitzerfläche S_a und Glitzerintensität S_i einer Nagellackapplikation enthaltend das Metalleffektpigment Visionaire Sparkling Silver Sea ohne Zusatz von mikronisiertem Wachs (Vergleichsbeispiel 3), in Gegenwart von Ceraflour 913 (Beispiel 3), Ceraflour 914 (Beispiel 7) und Talk (Vergleichsbeispiel 7) bei einer Messgeometrie von 45° (vermessen auf schwarzem Untergrund einer Schwarz-Weiß-Deckungskarte)

Abbildung 4: Glitzerfläche S_a und Glitzerintensität S_i einer Nagellackapplikation enthaltend das Metalleffektpigment Visionaire Sparkling Silver Sea ohne Zusatz von mikronisiertem Wachs (Vergleichsbeispiel 3), in Gegenwart von Ceraflour 913 (Beispiel 3), Ceraflour 914 (Beispiel 7) und Talk (Vergleichsbeispiel 7) bei einer Messgeometrie von 75° (vermessen auf schwarzem Untergrund einer Schwarz-Weiß-Deckungskarte)

Abbildung 5: Rasterelektronenmikroskopische Aufnahme einer Nagellackapplikation enthaltend das Metalleffektpigment Visionaire Sparkling Silver Sea (Vergleichsbeispiel 3)

10µm

Abbildung 6: Rasterelektronenmikroskopische Aufnahme einer Nagellackapplikation enthaltend das Metalleffektpigment Visionaire Sparkling Silver Sea (Vergleichsbeispiel 3)

20µm

Abbildung 7: Rasterelektronenmikroskopische Aufnahme einer Nagellackapplikation enthaltend das Metalleffektpigment Visionaire Sparkling Silver Sea und das mikronisierte Wachs Ceraflour 914 (Beispiel 7)

Abbildung 8: Rasterelektronenmikroskopische Aufnahme einer Nagellackapplikation enthaltend das Metalleffektpigment Visionaire Sparkling Silver Sea und das mikronisierte Wachs Ceraflour 914 (Beispiel 7)

Abbildung 9: Rasterelektronenmikroskopische Aufnahme einer Nagellackapplikation enthaltend das Metalleffektpigment Visionaire Sparkling Silver Sea und das mikronisierte Wachs Ceraflour 914 (Beispiel 7)

Abbildung 10: Lichtmikroskopisches Aufnahme (500-fache Vergrößerung) einer Nagellackapplikation enthaltend das Metalleffektpigment Visionaire Sparkling Silver Sea (Vergleichsbeispiel 3)

Abbildung 11: Lichtmikroskopisches Aufnahme (500-fache Vergrößerung) einer Nagellackapplikation enthaltend das Metalleffektpigment Visionaire Sparkling Silver Sea und das mikronisierte Wachs Ceraflour 913 (Beispiel 3)

Abbildung 12: Lichtmikroskopisches Aufnahme (500-fache Vergrößerung) einer Nagellackapplikation enthaltend das Metalleffektpigment Visionaire Sparkling Silver Sea und das mikronisierte Wachs Ceraflour 914 (Beispiel 7)

Abbildung 13: Lichtmikroskopisches Aufnahme (500-fache Vergrößerung) einer Nagellackapplikation enthaltend das Metalleffektpigment Visionaire Sparkling Silver Sea und Talk (Vergleichsbeispiel 7)

Abbildung 14: Rauwerte Rq von Nagellackapplikationen enthaltend das Metalleffektigment Visionaire Sparkling Silver Sea ohne Zusatz von mikronisiertem Wachs (Vergleichsbeispiel 3), in Gegenwart von Ceraflour 913 (Beispiel 3), Ceraflour 914 (Beispiel 7) nd Talk (Vergleichsbeispiel 7)

**Rauwerte Rq**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4822423 A
- US 20090126316 A1
- WO 2007039832 A2
- WO 2009097517 A1
- US 6367484 B1
- US 5612021 A
- US 4407310 A
- WO 0027347 A1
- DE 2345560
- EP 1532213 B1
- EP 1758550 A2
- EP 0848735 B1
- EP 1685198 B1
- EP 1251152 B1
- EP 0289240 A1
- WO 2004056716 A1
- WO 2005063637 A1
- EP 1980594 B1
- WO 2009103322 A1
- WO 200188044 A1

**In der Beschreibung aufgeführte Nicht-Patentliteratur**

- Römpp Lexikon Lacke und Druckfarben. Georg Thieme Verlag, 1998
- *Code of Federal Regulations*
- *CHEMICAL ABSTRACTS,* 1344-09-8
- *CHEMICAL ABSTRACTS,* 7775-19-1
- *CHEMICAL ABSTRACTS,* 7631-86-9
- **S. SCHELLENBERGER ; M. ENTENMANN ; A. HENNEMANN ; P. THOMETZEK.** *Farbe und Lack,* April 2007, 130
- **BYK-GARDNER.** *Qualitätskontrolle für Lacke und Kunststoffe,* 2011, 97, , 98
- **BYK-GARDNER.** *Qualitätskontrolle für Lacke und Kunststoffe,* 2011, 16